# EUROPEAN PATENT APPLICATION

(11) **EP 4 796 154 A1**
(43) Date of publication of application: **26.08.2026**
(21) Application number: 23955637.6
(22) Date of filing: 16.10.2023
(51) Int. Cl.: A61M 16/08, A61M 16/10, A61M 39/00, A61M 39/10

(54) **BREATHING TUBE, MEDICAL APPARATUS, AND PROCESSING METHOD**

(71) Applicant: Mehow Innovative Ltd., Shenzhen, Guangdong 518116 (CN)
(72) Inventor: WANG, Honghai, Shenzhen, Guangdong 518116 (CN); PAN, Xinming, Shenzhen, Guangdong 518116 (CN); ZHU, Han, Shenzhen, Guangdong 518116 (CN); ZHOU, Kunxing, Shenzhen, Guangdong 518116 (CN); YU, Wenbin, Shenzhen, Guangdong 518116 (CN); HU, Yalan, Shenzhen, Guangdong 518116 (CN)
(74) Representative: Santarelli
(86) International application number: PCT/CN2023/124759
(87) International publication number: WO 2025/081308

(57) **Abstract**

Provided are a breathing tube, a medical device, and a manufacturing method. The breathing tube includes a gas guide tube and a first support rib. The gas guide tube is configured to convey a gas. The first support rib is connected to an outer peripheral wall surface of the gas guide tube and disposed around an axis of the gas guide tube. The first support rib includes a hollow segment that is hollowed out. The first support rib that is partially hollowed out has lower thermal conductivity, so as to enhance the heat preservation and insulation effects for a flowing medium, thereby reducing the amount of condensate water caused by a temperature difference on the tube wall. **In** addition, the first support rib can effectively improve the support effect on the gas guide tube. Not only can poor flow of mediums in the breathing tube due to significant bending thereof be avoided, but also the material of the support rib can be saved, thereby reducing the production cost. The hollow structure of the hollow segment of the support rib enables the breathing tube to have a low weight while ensuring the same strength and is beneficial to improving the heat preservation and insulation performance.

## Description

### Technical Field

The present disclosure relates to the field of medical devices, and in particular, to a breathing tube, a medical device, and a manufacturing method.

### Background of the Invention

A ventilator is a medical device for assisting a patient's breathing. Its working principle involves compressing air or oxygen, and then delivering the gas that is regulated through a series of conduits and valves into the patient's respiratory system, thereby increasing oxygen supply or improving ventilation. A breathing tube is a conduit system connecting the ventilator and the patient. It includes a gas inlet conduit and a gas outlet conduit. The ventilator typically delivers the gas into the patient's respiratory system through these conduits and then returns the exhaled gas back to the ventilator.

In the related art, the breathing tube is mainly formed by a thin film. To avoid poor flow of the gas in the breathing tube due to bending thereof, a plurality of solid reinforcing rib strips are arranged circumferentially around the outer peripheral wall of the breathing tube, thereby providing effective support for the breathing tube. The medium within the breathing tube is prone to exchanging heat with the external environment through the outer wall of the breathing tube and the solid reinforcing rib strips. Consequently, the breathing tube has poor heat preservation and insulation performance, resulting in easy formation of condensate water on its inner wall, which can affect the quality of gas supply to the patient and, in severe cases, endanger the patient's life safety.

### Summary of the Invention

A main objective of the present disclosure is to provide a breathing tube, a medical device, and a manufacturing method to solve the technical problems of excessively high weight and poor heat preservation and insulation performance of the breathing tube with a rib strip structure.

To achieve the above objective, embodiments of a first aspect of the present disclosure propose a breathing tube, including:
a gas guide tube configured to convey a gas; and
a first support rib connected to an outer peripheral wall surface of the gas guide tube and disposed around an axis of the gas guide tube;
where the first support rib includes a hollow segment that is hollowed out.

In some embodiments, the first support rib is entirely hollowed out.

In some embodiments, the first support rib includes a plurality of annular rings that are sleeved outside the gas guide tube and are arranged at intervals along a direction of the axis of the gas guide tube;
or
the first support rib is spirally wound around the gas guide tube with the axis of the gas guide tube as a center axis.

In some embodiments, the first support rib is spirally wound around the gas guide tube with the axis of the gas guide tube as a center axis; and
a limiting protrusion is provided on the outer peripheral wall surface of the gas guide tube, and abuts against a spiral side edge of the first support rib along the direction of the axis of the gas guide tube.

In some embodiments, two limiting protrusions are provided on the outer peripheral wall surface of the gas guide tube, and abut against two opposite spiral side edges of the first support rib along the direction of the axis of the gas guide tube, respectively.

In some embodiments, the two limiting protrusions are spirally wound around the gas guide tube with the axis of the gas guide tube as the center axis.

In some embodiments, the hollow segment includes a ribbed tube connected to the outer peripheral wall surface and a rib strip disposed within the ribbed tube; a side of the rib strip close to the gas guide tube is connected to an inner wall surface of a side of the ribbed tube close to the gas guide tube, and a side of the rib strip away from the gas guide tube is connected to an inner wall surface of a side of the ribbed tube away from the gas guide tube.

In some embodiments, the rib strip is disposed spirally with the axis of the gas guide tube as a center axis.

In some embodiments, a thickness of the rib strip decreases gradually along a direction away from the gas guide tube.

In some embodiments, the hollow segment includes a plurality of rib strips that are spaced apart along a radial direction of the ribbed tube; and spacings between ribbed tubes increase gradually along a direction away from the gas guide tube.

In some embodiments, the gas guide tube is configured to be formed by spirally winding a strip.

In some embodiments, the strip includes a strip body and a lug; the strip body has an inner overlapping portion on one side and an outer overlapping portion on another side along a width direction of the strip body; the inner overlapping portion and the outer overlapping portion of the strip body in a spiral shape are arranged in an overlapping manner, and the outer overlapping portion is located on a side of the inner overlapping portion away from a lumen of the gas guide tube; and
the lug is connected to the outer overlapping portion and extends along a length direction of the strip body; and the lug in the spiral shape is a limiting protrusion.

In some embodiments, one lug is provided such that the gas guide tube includes one limiting protrusion; and along an axial direction of the gas guide tube, one side of the limiting protrusion abuts against a spiral side edge on one side of the first support rib, while another side of the limiting protrusion abuts against a spiral side edge on another side of the first support rib.

In some embodiments, two lugs are provided such that the gas guide tube includes two limiting protrusions; and the first support rib is located between the two limiting protrusions.

In some embodiments, the gas guide tube is made of a transparent material;
and/or
the first support rib is made of a transparent material.

In some embodiments, the breathing tube further includes a heating wire connected to at least one of the gas guide tube and the first support rib.

In some embodiments, the heating wire is disposed spirally with the axis of the gas guide tube as a center axis.

In some embodiments, the first support rib is spirally wound around the gas guide tube with the axis of the gas guide tube as a center axis; and
the heating wire is located between a lumen of the gas guide tube and an outer side portion of the first support rib away from the gas guide tube.

In some embodiments, the heating wire is located within the gas guide tube;
or
the heating wire is located between the gas guide tube and the first support rib;
   or
the heating wire is located within the first support rib.

In some embodiments, the first support rib is entirely hollowed out, and includes a first sidewall connected to the gas guide tube and a second sidewall away from the gas guide tube; the first sidewall and the second sidewall together define an inner cavity of the first support rib; and the heating wire is located within the first sidewall.

In some embodiments, two limiting protrusions are provided on the outer peripheral wall surface of the gas guide tube, and abut against two opposite spiral side edges of the first support rib along the direction of the axis of the gas guide tube, respectively; and
the heating wire is located between the two limiting protrusions along an axial direction of the gas guide tube.

In some embodiments, a plurality of heating wires are provided, and each of the plurality of heating wires is disposed spirally with the axis of the gas guide tube as a center axis;
or
a plurality of heating wires are provided, and each of the plurality of heating wires is disposed spirally with the axis of the gas guide tube as a center axis, with at least one of the plurality of heating wires being adapted to transfer a communication signal.

In some embodiments, the gas guide tube is configured to be formed by spirally winding a strip;
the strip includes a strip body; the strip body has an inner overlapping portion on one side and an outer overlapping portion on another side along a width direction of the strip body; the inner overlapping portion and the outer overlapping portion of the strip body in a spiral shape are arranged in an overlapping manner, and the outer overlapping portion is located on a side of the inner overlapping portion away from a lumen of the gas guide tube; and
the heating wire is located between the inner overlapping portion and the outer overlapping portion along a radial direction of the gas guide tube.

In some embodiments, the breathing tube further includes a second support rib connected to the outer peripheral wall surface of the gas guide tube and disposed around the axis of the gas guide tube.

In some embodiments, the second support rib is disposed spirally with the axis of the gas guide tube as a center axis; and the second support rib and the first support rib are of a dual-spiral structure.

In some embodiments, the second support rib is entirely hollowed out;
or
the second support rib and an outer peripheral wall of the gas guide tube together define a spiral cavity.

In some embodiments, a height dimension of the second support rib is smaller than a height dimension of the first support rib along the radial direction of the gas guide tube;
and/or
a width dimension of the second support rib is smaller than a width dimension of the first support rib along an axial direction of the gas guide tube.

In some embodiments, the first support rib and the gas guide tube are integrated;
and/or
the second support rib and the gas guide tube are integrated.

In some embodiments, the second support rib and the gas guide tube are integrated;
the gas guide tube is configured to be formed by spirally winding a strip;
the strip includes a strip body and a support strip; the support strip is connected to a side of the strip body, and defines, together with the strip body, an inner cavity extending along a length direction of the strip; and
the support strip is disposed spirally to form the second support rib.

In some embodiments, the strip further includes a lug that is connected to the strip body and located on a same side with the support strip; the lug extends along a length direction of the strip body and is spaced apart from the support strip along a width direction of the strip body; and
the lug is disposed spirally to form a limiting protrusion; and the first support rib is located between the limiting protrusion and the second support rib.

In some embodiments, the strip further includes two lugs that are connected to the strip body and located on a same side with the support strip; the two lugs extend along a length direction of the strip body and are spaced apart from each other along a width direction of the strip body; and
the two lugs are disposed spirally to form two limiting protrusions; and the first support rib is located between the two limiting protrusions.

In some embodiments, the first support rib and the gas guide tube are integrated;
the gas guide tube is configured to be formed by spirally winding a strip;
the strip includes a strip body and a support strip; the support strip is connected to a side of the strip body, and defines, together with the strip body, an inner cavity extending along a length direction of the strip; and
the support strip is disposed spirally to form the first support rib.

In some embodiments, the breathing tube meets at least one of conditions a) to k):
a) a wall thickness d1 of the gas guide tube meets: 0.13 mm≤d1≤0.23 mm;
b) a wall thickness d2 of the hollow segment meets: 0.1 mm≤d2≤0.5 mm;
c) an inner diameter D1 of the gas guide tube meets: 8 mm≤D1≤28 mm;
d) along a radial direction of the gas guide tube, a height dimension L1 of the hollow segment meets: 2.2 mm≤L1≤2.8 mm;
e) along an axial direction of the gas guide tube, a width dimension L2 of the hollow segment meets: 2 mm≤L2≤6 mm;
f) the hollow segment is arranged spirally with the axis of the gas guide tube as a center axis, and a lead S of the hollow segment meets: 2 mm≤S≤8 mm;
g) the first support rib is spirally wound around the gas guide tube with the axis of the gas guide tube as the center axis; a limiting protrusion is provided on the outer peripheral wall surface of the gas guide tube, and abuts against a spiral side edge of the first support rib along a direction of the axis of the gas guide tube; and along the radial direction of the gas guide tube, a height dimension L₃ of the limiting protrusion meets: 0.3 mm≤L₃≤1.3 mm;
h) the first support rib is spirally wound around the gas guide tube with the axis of the gas guide tube as the center axis; a limiting protrusion is provided on the outer peripheral wall surface of the gas guide tube, and abuts against a spiral side edge of the first support rib along the direction of the axis of the gas guide tube; and along the axial direction of the gas guide tube, a width dimension L₄ of the limiting protrusion meets: 0.5 mm≤L₄≤1.5 mm;
i) the first support rib is arranged spirally with the axis of the gas guide tube as the center axis, and a lead of the first support rib is greater than a width dimension of the first support rib along the axial direction of the gas guide tube;
j) a second support rib is further wound around the gas guide tube; and along the radial direction of the gas guide tube, a height dimension L₅ of the second support rib meets: 1.3 mm≤L₅≤2.3 mm; and
k) a second support rib is further wound around the gas guide tube; and along the axial direction of the gas guide tube, a width dimension L₆ of the second support rib meets: 1.5 mm≤L₆≤2.5 mm.

Embodiments of a second aspect of the present disclosure further provide a breathing tube, including:
a gas guide tube configured to convey a gas; and
a first support rib connected to an outer peripheral wall surface of the gas guide tube and disposed around an axis of the outer peripheral wall surface;
where the first support rib includes a hollow segment; and the hollow segment and the outer peripheral wall surface of the gas guide tube together define a cavity.

Embodiments of a third aspect of the present disclosure further provide a medical device, including the breathing tube according to any one of above embodiments.

Embodiments of a fourth aspect of the present disclosure further provide a manufacturing method, used for manufacturing the breathing tube according to any one of above embodiments and including steps of:
fabricating the strip;
fabricating the support strip, where the support strip includes the hollow segment;
spirally winding the strip around a forming shaft to form the gas guide tube; and
spirally winding the support strip around the gas guide tube to form the first support rib.

In some embodiments, the step of fabricating the strip includes: forming the strip through an extrusion molding process using a first extrusion unit;
and/or
the step of fabricating the support strip includes: forming the support strip through an extrusion molding process using a second extrusion unit.

In some embodiments, the step of spirally winding the strip around a forming shaft to form the gas guide tube includes: disposing a first extrusion unit beside the forming shaft, and winding the formed strip around the forming shaft during a process of forming the strip by the first extrusion unit; and
the step of spirally winding the support strip around the gas guide tube to form the first support rib includes: disposing a second extrusion unit beside the forming shaft, and winding the formed support strip around the formed gas guide tube during a process of forming the support strip by the second extrusion unit.

In some embodiments, the step of spirally winding the strip around a forming shaft to form the gas guide tube further includes: disposing a plurality of flexible spring shafts along a circumferential direction of the forming shaft, spirally winding the strip around the flexible spring shafts, and driving, by a driver, the flexible spring shafts to move along an axial direction of the forming shaft and to rotate along the circumferential direction of the forming shaft, thereby driving the strip to move spirally.

In some embodiments, the step of fabricating the strip includes: integrally forming the heating wire and the strip through extrusion molding, where the heating wire is located within the strip;
or
the step of fabricating the support strip includes: integrally forming the heating wire and the support strip through extrusion molding, where the heating wire is located within the support strip;
   or
before the step of spirally winding the support strip around the gas guide tube to form the first support rib, the method including: spirally winding the heating wire around the gas guide tube, spirally winding the support strip around the gas guide tube, and covering the heating wire with the support strip.

In some embodiments, the step of fabricating the support strip includes: fabricating two support strips, where at least one of the two support strips includes the hollow segment; and
the step of spirally winding the support strip around the gas guide tube includes: spirally winding the two support strips around the gas guide tube to form the dual-spiral structure, thereby forming the first support rib and the second support rib.

Compared with the prior art, the present disclosure has the following beneficial effects:

In the technical solutions of the present disclosure, the breathing tube includes the gas guide tube and the first support rib. The first support rib is connected to the outer peripheral wall surface of the gas guide tube and is spirally wound around the gas guide tube with the axis of the gas guide tube as the center axis. The first support rib includes the hollow segment that is hollowed out. Compared with existing solutions in which breathing tubes are provided with solid support ribs, the first support rib that is partially hollowed out in the present disclosure has lower thermal conductivity, so as to effectively inhibit heat exchange between the flowing medium in the tube and the external environment and enhance the heat preservation and insulation effects for the flowing medium, thereby reducing the amount of condensate water caused by a temperature difference on the tube wall. Compared with an existing solution in which the breathing tube is provided with a single solid support rib, the first support rib that is partially hollowed out in the present disclosure can effectively improve the support effect on the gas guide tube. Not only can poor flow of mediums in the breathing tube due to significant bending thereof be avoided, but also the material of the support rib can be saved, thereby reducing the production cost. Thus, the hollow structure of the hollow segment of the support rib enables the breathing tube of the present disclosure to have a low weight while ensuring the same strength and is beneficial to improving the heat preservation and insulation performance.

### Brief Description of the Drawings

To explain the technical solutions in the embodiments of the present disclosure or in the prior art more clearly, the accompanying drawings required for describing the embodiments or the prior art will be described below briefly. Obviously, the accompanying drawings in the following description show merely some embodiments of the present disclosure, and other drawings can also be derived from structures illustrated in these accompanying drawings by a person of ordinary skill in the art without creative efforts.
FIG. 1 is a cross-sectional schematic diagram of a first support rib of a breathing tube according to an embodiment of the present disclosure, where one portion of the first support rib is a hollow segment, while the other portion is a solid segment;
FIG. 2 is a cross-sectional schematic diagram of a first support rib of a breathing tube according to an embodiment of the present disclosure, where the first support rib is completely a hollow segment;
FIG. 3 is a cross-sectional schematic diagram of a breathing tube according to an embodiment of the present disclosure, where a first support rib and an outer peripheral wall surface of a gas guide tube together define a cavity;
FIG. 4 is a cross-sectional schematic diagram of a breathing tube according to an embodiment of the present disclosure, where the breathing tube includes a limiting protrusion;
FIG. 5 is a partial cross-sectional schematic diagram of a breathing tube according to an embodiment of the present disclosure, where a limiting protrusion is provided on each side of a first support rib;
FIG. 6 is a partially enlarged schematic diagram of a part around the first support rib at position A in FIG. 5;
FIG. 7 is a partially enlarged schematic diagram of a part around a first support rib according to an embodiment of the present disclosure;
FIG. 8 is a partially enlarged schematic diagram of a part around a first support rib according to an embodiment of the present disclosure;
FIG. 9 is a partially enlarged schematic diagram of a part around a first support rib according to an embodiment of the present disclosure;
FIG. 10 is a partial cross-sectional schematic diagram of a strip and a first support rib of a breathing tube being wound completely according to an embodiment of the present disclosure, where the strip is wound layer by layer;
FIG. 11 is an exploded schematic diagram of an independent winding unit of the strip and the first support rib in FIG. 10;
FIG. 12 is a partial cross-sectional schematic diagram of a breathing tube according to an embodiment of the present disclosure, where the breathing tube includes a heating wire;
FIG. 13 illustrates an opposite arrangement form of a first support rib and a heating wire according to an embodiment of the present disclosure;
FIG. 14 illustrates an opposite arrangement form of a first support rib and a heating wire according to an embodiment of the present disclosure;
FIG. 15 illustrates an opposite arrangement form of a first support rib and a heating wire according to an embodiment of the present disclosure;
FIG. 16 is a partial cross-sectional schematic diagram of a breathing tube according to an embodiment of the present disclosure, where the breathing tube includes a heating wire;
FIG. 17 is a partially enlarged schematic diagram of area B in FIG. 16, where the heating wire is disposed between an inner overlapping portion and an outer overlapping portion;
FIG. 18 is a cross-sectional schematic diagram of the adjacently overlapping inner overlapping portion and outer overlapping portion of the strip in FIG. 16 being separated;
FIG. 19 is a partial cross-sectional schematic diagram of a breathing tube according to an embodiment of the present disclosure, where the breathing tube includes a second support rib;
FIG. 20 is a partially enlarged schematic diagram of area D in FIG. 19;
FIG. 21 is a cross-sectional schematic diagram of a first support rib and a lug of a breathing tube being wound around a strip according to an embodiment of the present disclosure;
FIG. 22 is a cross-sectional schematic diagram of a first support rib and a lug of a breathing tube being wound around a strip according to an embodiment of the present disclosure, where the strip is formed by overlapping and winding an inner overlapping portion and an outer overlapping portion;
FIG. 23 is a cross-sectional schematic diagram of a first support rib, a lug, and a strip of a breathing tube being integrated according to an embodiment of the present disclosure;
FIG. 24 is a cross-sectional schematic diagram of a first support rib, a lug, and a strip of a breathing tube being wound according to an embodiment of the present disclosure;
FIG. 25 is a cross-sectional schematic diagram of a first support rib, a lug, and a strip of a breathing tube being integrated according to an embodiment of the present disclosure;
FIG. 26 is a schematic diagram of a first extrusion unit, a second extrusion unit, and a forming shaft according to an embodiment of the present disclosure;
FIG. 27 is a flowchart of a manufacturing method according to an embodiment of the present disclosure;
FIG. 28 is a flowchart of a first part of another manufacturing method according to an embodiment of the present disclosure; and
FIG. 29 is a flowchart of a second part of another manufacturing method according to an embodiment of the present disclosure, where S205 is a step behind S204 in FIG. 28, and the two steps are adjacent.

### List of Reference Numerals:

10-breathing tube;
100-gas guide tube; 110-outer peripheral wall surface; 120-axis;
130-strip; 131-strip body; 1311-inner overlapping portion; 1312-outer overlapping portion; 132-support strip; 133-lug;
200-support rib;
200a-first support rib; 210a-first sidewall; 220a-second sidewall; 230a-spiral side edge; 240a-ribbed tube; 250a-rib strip;
200b-second support rib;
300-limiting protrusion;
400-heating wire;
500-hollow segment;
610-first extrusion unit; 620-second extrusion unit; 630-forming shaft;
X-length direction; and
Y-width direction.

The implementation of the objective, functional characteristics, and advantages of the present disclosure will be further described in conjunction with the embodiments and with reference to the accompanying drawings.

### Detailed Description of Embodiments

The following clearly and completely describes the technical solutions in the embodiments of the present disclosure with reference to the accompanying drawings in the embodiments of the present disclosure. Apparently, the described embodiments are merely a part rather than all of the embodiments of the present disclosure. All other embodiments obtained by those of ordinary skill in the art based on the embodiments of the present disclosure without creative efforts should fall within the protection scope of the present disclosure.

A ventilator is a medical device for assisting or replacing a patient's breathing. Its working principle involves compressing air or oxygen, and then delivering the gas that is regulated through a series of conduits and valves into the patient's respiratory system, thereby increasing oxygen supply or improving ventilation. A breathing tube is a conduit system connecting the ventilator and the patient. It includes a gas inlet conduit and a gas outlet conduit. The ventilator typically delivers the gas into the patient's respiratory system through these conduits and then returns the exhaled gas back to the ventilator.

In the related art, the breathing tube is mainly formed by a thin film. To avoid poor flow of the gas in the breathing tube due to bending thereof, a plurality of solid reinforcing rib strips are arranged circumferentially around the outer peripheral wall of the breathing tube, thereby providing effective support for the breathing tube. The medium within the breathing tube is prone to exchanging heat with the external environment through the outer wall of the breathing tube and the solid reinforcing rib strips. Consequently, the breathing tube has poor heat preservation and insulation performance, which can affect the quality of gas supply to the patient.

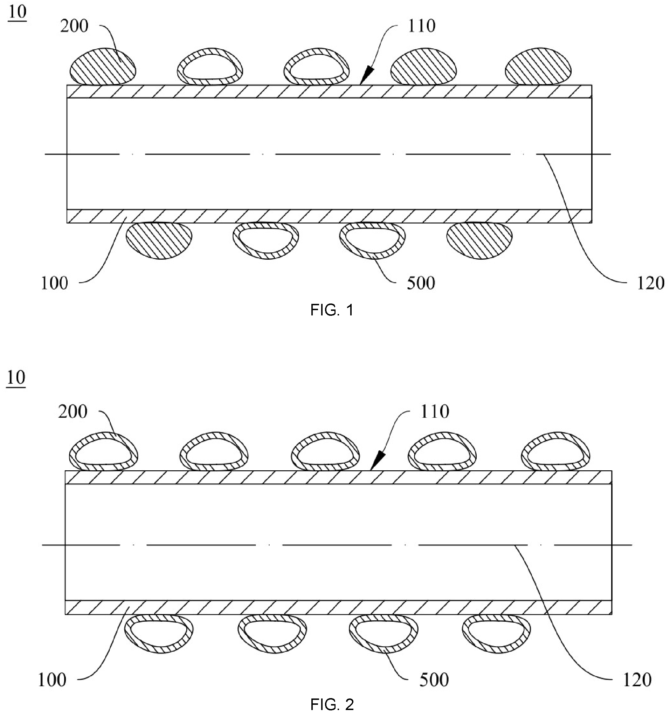

In view of this, an embodiment of the present disclosure provides a breathing tube 10. The breathing tube 10 is light in weight, can reduce the occurrence of disconnection between the breathing tube 10 and the patient, and has good heat preservation and insulation performance. The breathing tube 10 of this embodiment of the present disclosure is described below with reference to FIG. 1 to FIG. 26. Specifically, the breathing tube 10 includes a gas guide tube 100 and a first support rib 200a.

The gas guide tube 100 is configured to convey a gas. The gas may include oxygen, so that the gas guide tube 100 can provide breathing support for a patient. In some embodiments, the gas guide tube 100 may be a gas inlet conduit of the breathing tube 10. That is, the gas guide tube 100 may convey oxygen or air into the patient's lungs. In some other embodiments, the gas guide tube 100 may alternatively be a gas outlet conduit of the breathing tube 10. That is, the gas guide tube 100 may discharge the patient's exhaled gas. This depends on the actual situations.

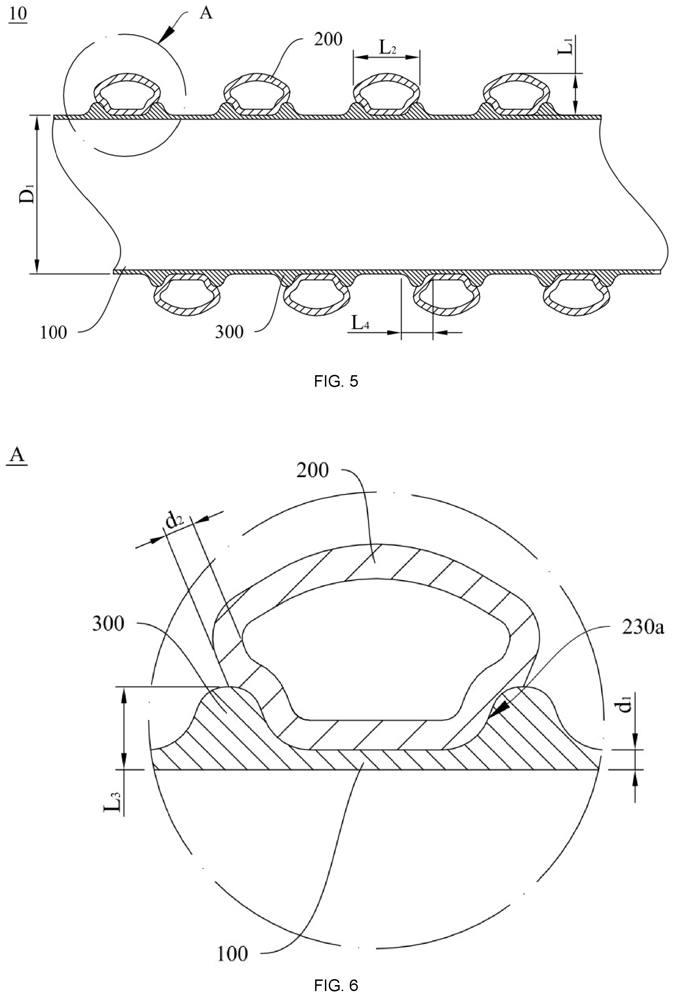

With reference to FIG. 1 to FIG. 6, the first support rib 200a is connected to an outer peripheral wall surface 110 of the gas guide tube 100. Regarding the connection form of the first support rib 200a and the gas guide tube 100, with reference to FIG. 1 to FIG. 6, in some embodiments, the first support rib 200a may be spirally wound around the gas guide tube 100 with an axis 120 of the gas guide tube as a center axis. The spiral winding direction and the pitch of the first support rib 200a may depend on the actual situations, and in different embodiments, the value of each pitch formed by each turn of the first support rib 200a may be identical or different. It will be understood that a helix may be a left-hand helix or a right-hand helix. According to this solution, the support rib 200 is arranged to be spirally wound around the gas guide tube 100, so that the stability of support of the support rib 200 for the gas guide tube 100 can be further improved, thereby preventing the gas guide tube 100 from bending significantly. Further, the occurrence of foreign objects directly scratching the gas guide tube 100 is reduced. Besides, it is ensured that the gas guide tube 100 can convey a flowing medium continuously and normally. Furthermore, specifically, the pitch of the first support rib 200a (or the pitch between the first support rib 200a and another support rib 200 adjacent thereto) is greater than the width of two first support ribs 200a. Preferably, the pitch of the first support rib 200a is greater than the width of three first support ribs 200a. It should be noted that the width of the first support rib 200a is explained as follows. With reference to FIG. 5 and FIG. 6, the breathing tube 10 is cut along a section plane where the axis 120 of the gas guide tube 100 lies. The support rib 200 and the peripheral wall of the gas guide tube 100 together form a plurality of closed shapes. The maximum width of the outermost line of a single closed shape along the direction of the axis 120 of the gas guide tube 100 is defined as the width of the first support rib 200a.

In some other embodiments, the first support rib 200a may be annularly disposed around the gas guide tube 100. Specifically, the first support rib 200a may include a plurality of annular rings. The specific number of annular rings depends on the specific length of the gas guide tube 100, a preset spacing, and the like. The annular rings are sleeved outside the gas guide tube 100 and are arranged at intervals along the direction of the axis 120 of the gas guide tube 100. It will be understood that the spacings between the annular rings may be uniform or non-uniform. In some embodiments of the present disclosure, uniform spacing between adjacent annular rings is taken as an example for illustration. It will be understood that in some embodiments, the annular rings may be arranged at intervals in a direction perpendicular to the axis 120 of the gas guide tube 100. In some other embodiments, the annular rings may be arranged at intervals in a direction inclined relative to the axis 120 of the gas guide tube 100. This depends on the actual situations. It should be noted that the annular rings may be of the same size or different sizes. In some embodiments of the present disclosure, the annular rings are described as having the same size by way of example. By providing a plurality of annular rings, this solution achieves stable support for the gas guide tube 100 and prevents significant bending of the gas guide tube 100, which would otherwise cause poor gas flow.

To enable the support rib 200 to provide better support and to increase the winding manufacturing speed, in some embodiments, a plurality of support ribs 200 may be provided. Specifically, in some embodiments, the breathing tube 10 may further include a second support rib 200b. The second support rib 200b may be connected to the outer peripheral wall surface 110 of the gas guide tube 100 and disposed around the axis 120 of the gas guide tube 100. Further, the second support rib 200b may be disposed spirally with the axis 120 of the gas guide tube 100 as a center axis; and the second support rib 200b and the support rib 200 are of a dual-spiral structure. The second support rib 200b may be entirely hollowed out. Alternatively, the second support rib 200b and the outer peripheral wall of the gas guide tube 100 may together define a spiral cavity. For ease of description, an embodiment in which the breathing tube 10 includes both the first support rib 200a and the second support rib 200b and the first support rib 200a and the second support rib 200b form a dual-spiral structure is described below as an example. Different embodiments may be combined with one another among different technical solutions, and may be further combined to obtain an embodiment in which only the first support rib 200a is disposed in a single-spiral configuration.

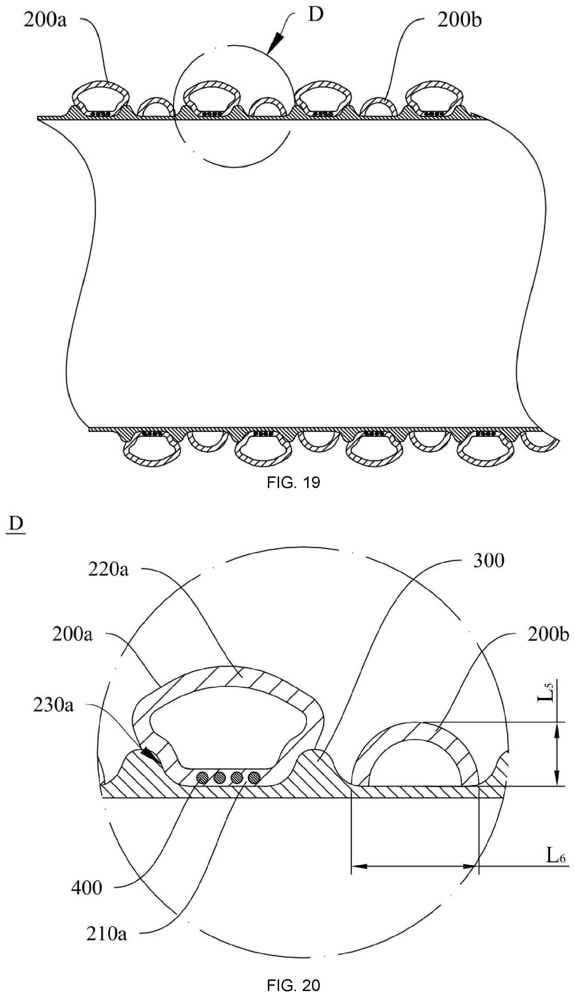

Based on the arrangement of the second support rib 200b in the above embodiments, similarly, with reference to FIG. 19, the second support rib 200b may be connected to the outer peripheral wall surface 110 of the gas guide tube 100, and the second support rib 200b may be spirally wound around the gas guide tube 100 with the axis 120 of the gas guide tube as the center axis. It will be understood that the second support rib 200b and the first support rib 200a may be spirally wound around the gas guide tube 100 in the same or similar form. Therefore, the structure and winding form of the second support rib 200b may refer to those of the first support rib 200a described above. In different embodiments, the structure or winding form may be the same or different for the second support rib 200b and the first support rib 200a. In different embodiments, the first support rib 200a or the second support rib 200b may be partially spirally wound around the axis 120 of the gas guide tube 100, or may be fully spirally wound around the axis 120 of the gas guide tube 100. In some embodiments of the present disclosure, both the first support rib 200a and the second support rib 200b are described as being fully spirally wound around the axis 120 of the gas guide tube 100 by way of example. Based on the limitations of the above embodiments, both the first support rib 200a and the second support rib 200b may be configured to support the gas guide tube 100. Furthermore, in some embodiments, the second support rib 200b may be entirely hollowed out. Alternatively, the second support rib 200b and the outer peripheral wall of the gas guide tube 100 together define a spiral cavity.

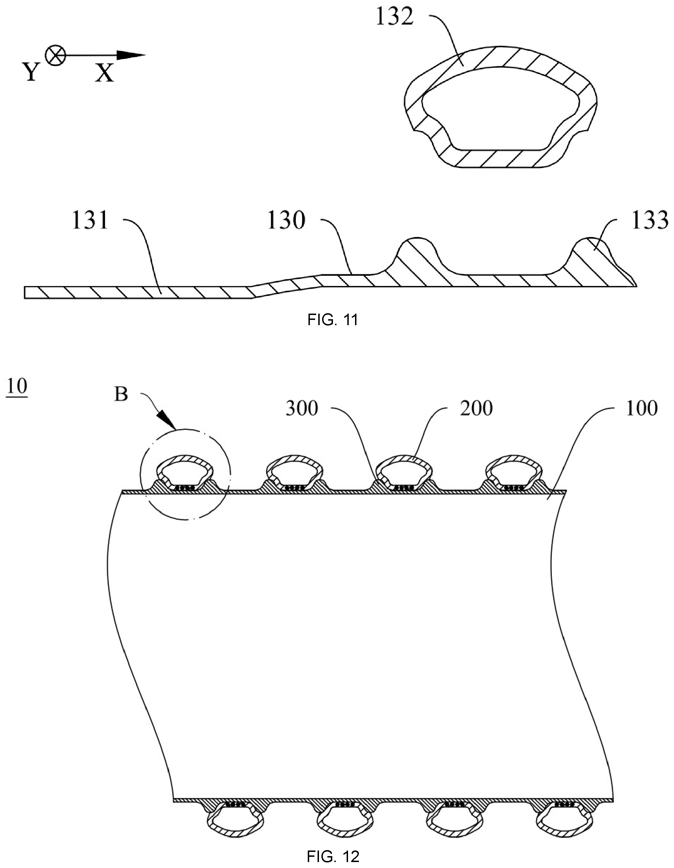

To make the gas delivered in the breathing tube 10 suitable for use by the patient, the breathing tube 10 may further include a heating wire 400. The heating wire 400 may be directly or indirectly connected to the gas guide tube 100 in any form. With reference to FIG. 12, in some embodiments, the heating wire 400 may be disposed spirally with the axis 120 of the gas guide tube 100 as the center axis. It will be understood that the heating wire 400 may be configured to heat the gas delivered within the gas guide tube 100, and the winding arrangement of the heating wire 400 allows the heating wire 400 to have a larger contact area with the outer peripheral wall surface 110 of the gas guide tube 100, thereby helping to improve the heating efficiency. To facilitate signal transfer to the heating wire 400 for controlling the heating of the heating wire 400, in some embodiments, the breathing tube 10 may further include a conductive wire. The conductive wire may be electrically connected to the heating wire 400, and the conductive wire may also be disposed spirally with the axis 120 of the gas guide tube 100 as the center axis. The aforementioned conductive wire may be adapted to transfer a communication signal. In some other embodiments, a plurality of heating wires 400 may be provided, and each heating wire 400 may be disposed spirally with the axis 120 of the gas guide tube 100 as the center axis. At least one heating wire 400 may be adapted to transfer a communication signal. The specific arrangement of the heating wire 400 will be described below. For ease of description, an embodiment in which the breathing tube 10 includes the heating wire 400 and the heating wire 400 is disposed spirally with the axis 120 of the gas guide tube 100 as the center axis is described below as an example. Different embodiments may be combined with one another among different technical solutions.

In particular, the first support rib 200a includes a hollow segment 500. The hollow segment 500 is hollowed out. It will be understood that, with reference to FIG. 1, in some embodiments, one portion of the first support rib 200a may be hollowed out (this portion is the hollow segment 500), while the other portion thereof may be solid. In some other embodiments, with reference to FIG. 2, the first support rib 200a may be entirely hollowed out. That is, the entire first support rib 200a may be formed as the hollow segment 500. The position and distribution of the hollow segment 500 may vary freely according to usage requirements. A single cross-section of the first support rib 200a along the direction perpendicular to the axis 120 of the gas guide tube 100 may include both a hollow segment 500 and a non-hollow segment 500. For example, one side of the single cross-section of the first support rib 200a along the direction perpendicular to the axis 120 of the gas guide tube 100 may be the hollow segment 500, and another side may be a solid segment. The above-described segmented arrangement of the first support rib 200a allows the solid portion of the breathing tube 10 to provide relatively high supporting strength, while the hollow segment 500 enables the breathing tube 10 to have a lower weight and better heat preservation and insulation performance. The entirely hollow configuration of the first support rib 200a can significantly reduce the weight of the breathing tube 10 and provide even better heat preservation and insulation performance. For ease of description, an embodiment in which the first support rib 200a is entirely hollowed out is described below as an example. Different embodiments may be combined with one another among different technical solutions.

Based on the above arrangement, the wall thickness of the first support rib 200a may be determined according to the actual situations. With reference to the practical application scenarios of the breathing tube 10, it will be understood that compared with existing solid ribbed plate structures, the hollow configuration of the first support rib 200a achieves a lower weight while meeting the same strength requirements (alternatively, it can be understood as meeting higher strength requirements while having the same weight). The first support rib 200a and the second support rib 200b form a dual-spiral structure.

It should be noted that, in different embodiments, the first support rib 200a may be entirely hollowed out or partially hollowed out. In other words, depending on the strength and thermal insulation requirements of the breathing tube 10 as well as the arrangement of the heating wire 400, in some embodiments, the first support rib 200a may be configured such that only a portion of its winding segments is hollow. For example, the hollow first support rib 200a may be provided in the portion where the heating wire 400 is to be disposed. It will be understood that the above-described partial hollow configuration helps reduce the manufacturing cost, and the portion of the first support rib 200a that is not hollowed out may be configured as a solid structure, thereby further enhancing the supporting strength of the first support rib 200a. For ease of description, an embodiment in which the first support rib 200a may be entirely hollowed out is described below as an example. Different embodiments may be combined with one another among different technical solutions. Furthermore, in some embodiments, the second support rib 200b may be entirely hollowed out. Alternatively, the second support rib 200b and the outer peripheral wall of the gas guide tube 100 together define a spiral cavity.

In the technical solutions of the present disclosure, the breathing tube 10 includes the gas guide tube 100 and the first support rib 200a. The first support rib 200a is connected to the outer peripheral wall surface 110 of the gas guide tube 100. The first support rib 200a is spirally disposed around the gas guide tube 100 with the axis 120 of the gas guide tube 100 as the center axis. The first support rib 200a includes a hollow segment 500. The hollow segment 500 is hollowed out. Compared with existing solutions in which breathing tubes 10 are provided with solid support ribs 200, the first support rib 200a that is partially hollowed out in the present disclosure has lower thermal conductivity, so as to effectively inhibit heat exchange between the flowing medium in the tube and the external environment and enhance the heat preservation and insulation effects for the flowing medium, thereby reducing the amount of condensate water caused by a temperature difference on the tube wall. Compared with an existing solution in which the breathing tube 10 is provided with a single solid support rib 200, the first support rib 200a that is partially hollowed out in the present disclosure can effectively improve the support effect on the gas guide tube 100. Not only can poor flow of mediums in the breathing tube 10 due to significant bending thereof be avoided, but also the material of the support rib 200 can be saved, thereby reducing the production cost. Thus, the hollow structure of the hollow segment 500 of the support rib 200 enables the breathing tube 10 of the present disclosure to have a low weight while ensuring the same strength and is beneficial to improving the heat preservation and insulation performance.

In embodiments including both the first support rib 200a and the second support rib 200b, the first support rib 200a and the second support rib 200b may have different structural forms. Specifically, on the one hand, with reference to FIG. 19 and FIG. 20, regarding the height dimensions of the first support rib 200a and the second support rib 200b, in some embodiments, the height dimension of the second support rib 200b is smaller than that of the first support rib 200a along the radial direction of the gas guide tube 100. The height dimension of the first support rib 200a is defined as a distance, along the direction perpendicular to the axis 120 of the gas guide tube 100, from the end of the first support rib 200a close to the axis 120 of the gas guide tube 100 to the end of the first support rib 200a away from the axis 120 of the gas guide tube 100. Similarly, the height dimension of the second support rib 200b is defined as a distance, along the direction perpendicular to the axis 120 of the gas guide tube 100, from the end of the second support rib 200b close to the axis 120 of the gas guide tube 100 to the end of the second support rib 200b away from the axis 120 of the gas guide tube 100. It will be understood that the above height definition means that, along the direction perpendicular to the axis 120 of the gas guide tube 100, the height of the first support rib 200a is greater than that of the second support rib 200b. This arrangement can reduce the manufacturing cost of the breathing tube 10 and lower the weight of the breathing tube 10.

On the other hand, with reference to FIG. 19 and FIG. 20, regarding the width dimensions of the first support rib 200a and the second support rib 200b, in some embodiments, the width dimension of the second support rib 200b is smaller than that of the first support rib 200a along the axial direction of the gas guide tube 100. The width dimension of the first support rib 200a is defined as a distance from one end of the first support rib 200a along the direction of the axis 120 of the gas guide tube 100 to the opposite end thereof. Similarly, the width dimension of the second support rib 200b is defined as a distance from one end of the second support rib 200b along the direction of the axis 120 of the gas guide tube 100 to the opposite end thereof. It will be understood that the above width definition means that, along the direction of the axis 120 of the gas guide tube 100, the width of the first support rib 200a is greater than that of the second support rib 200b. This arrangement can reduce the manufacturing cost of the breathing tube 10 and lower the weight of the breathing tube 10, and also facilitate controlling and adjusting the distance between the first support rib 200a and the second support rib 200b.

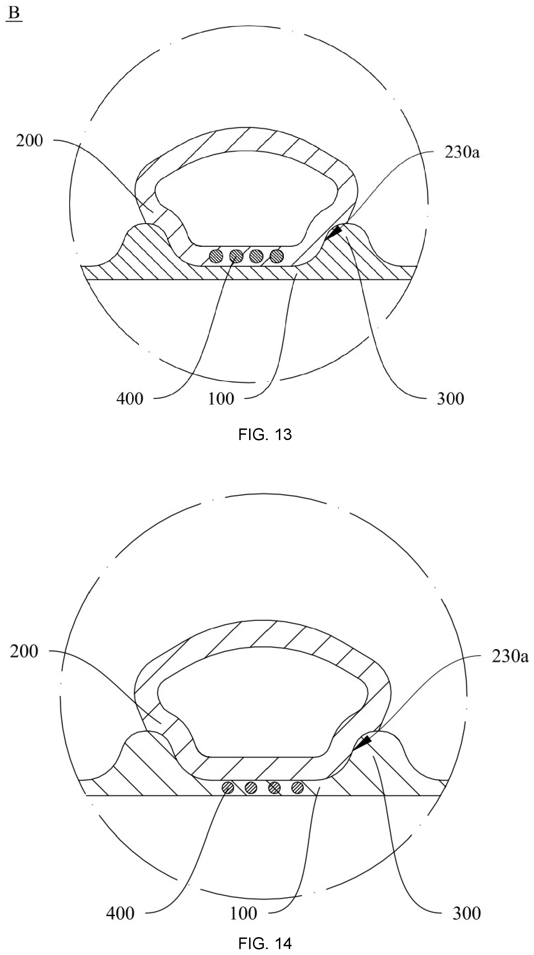

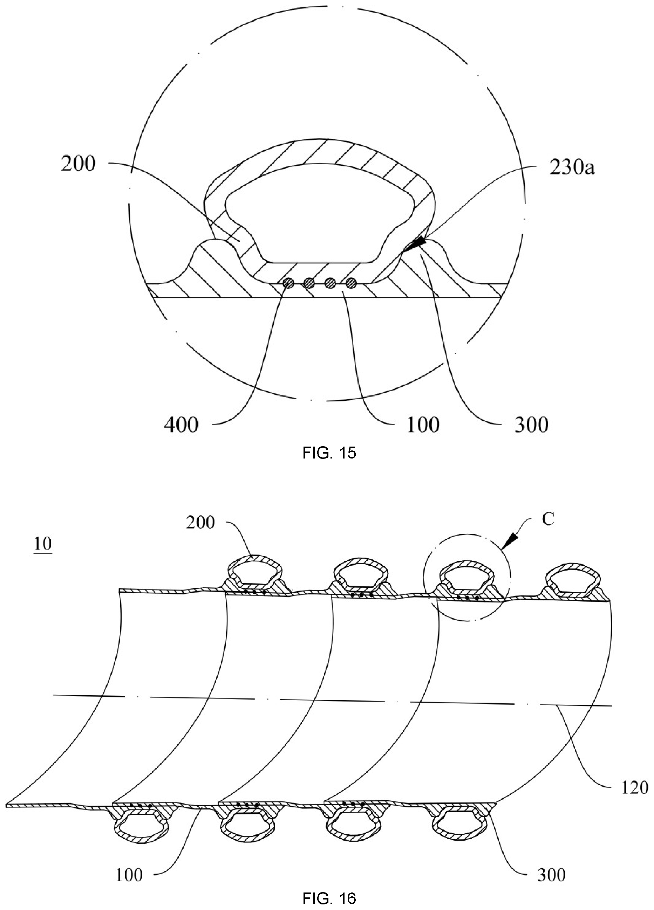

In different embodiments, the heating wire 400 may be arranged at different positions. To fixedly connect the heating wire 400 and enable the heating wire 400 to have a good heating effect, in some embodiments, the heating wire 400 may be connected to at least one of the gas guide tube 100 and the first support rib 200a. Regarding the specific positional relationship of the heating wire 400 with respect to the gas guide tube 100 and the first support rib 200a, with reference to FIG. 14, in an embodiment of a first arrangement of the heating wire 400, the heating wire 400 may be located within the gas guide tube 100. Specifically, in some embodiments, the heating wire 400 may be connected to the inner wall surface of the gas guide tube 100. The heating wire 400 may be connected inside the gas guide tube 100 via bonding, snap-in connection, or the like. The above arrangement allows the heating wire 400 to be positioned closer to the gas flowing inside the gas guide tube 100, thereby helping to improve the heating efficiency of the heating wire 400 for the gas inside the gas guide tube 100. With reference to FIG. 15, in an embodiment of a second arrangement of the heating wire 400, the heating wire 400 may be located between the gas guide tube 100 and the first support rib 200a. It will be understood that, along the direction perpendicular to the axis 120 of the gas guide tube 100, the heating wire 400 may be sandwiched between the gas guide tube 100 and the first support rib 200a, so that the gas guide tube 100 and the first support rib 200a can together clamp the heating wire 400, thereby achieving higher heating efficiency of the heating wire 400 for the gas inside the gas guide tube 100 and better heat preservation and insulation effect of the first support rib 200a. For embodiments in which the first support rib 200a itself is arranged in a layered and wound manner, the heating wire 400 may alternatively be located between two adjacent turns of the wound first support rib 200a. This specific form will be described later. With reference to FIG. 13, in an embodiment of a third arrangement of the heating wire 400, the heating wire 400 may be located within the first support rib 200a. Specifically, in some embodiments, the first support rib 200a may include a first sidewall 210a connected to the gas guide tube 100 and a second sidewall 220a away from the gas guide tube 100. The first sidewall 210a and the second sidewall 220a together define an inner cavity of the first support rib 200a. It will be understood that the first sidewall 210a and the second sidewall 220a are arranged opposite to each other along the direction perpendicular to the axis 120 of the gas guide tube 100, and the first sidewall 210a close to the axis 120 of the gas guide tube 100 is connected to the gas guide tube 100. Based on this, the heating wire 400 may be located within the first sidewall 210a. The above arrangement allows the heating wire 400 to pass through the wall thickness portion of the first sidewall 210a, so that the heating wire 400 can be spirally wound around the gas guide tube 100 together with the first support rib 200a. This helps reduce the manufacturing time and also enables a more secure connection of the heating wire 400.

Regarding the connection relationship of both the first support rib 200a and the second support rib 200b with the outer peripheral wall surface 110 of the gas guide tube 100, in some embodiments, the first support rib 200a or the second support rib 200b may be a separately manufactured component. That is, the gas guide tube 100 and the first support rib 200a or the second support rib 200b may be provided separately, and then assembled together. Therefore, in such embodiments, the first support rib 200a or the second support rib 200b may be connected to the gas guide tube 100 in a detachable (or separable) manner. Specifically, the first support rib 200a or the second support rib 200b may be connected to the outer peripheral wall surface 110 by winding force, or may be bonded to the outer peripheral wall surface 110. The above arrangement facilitates adjustment and replacement of the first support rib 200a or the second support rib 200b. Correspondingly, in some other embodiments, the first support rib 200a may be integrated with the gas guide tube 100; and/or the second support rib 200b may be integrated with the gas guide tube 100. Thus, in such embodiments, the first support rib 200a or the second support rib 200b may be integrally formed with the gas guide tube 100. The above arrangement facilitates saving the manufacturing time of the breathing tube 10, and the integral configuration allows the first support rib 200a or the second support rib 200b to have higher supporting strength.

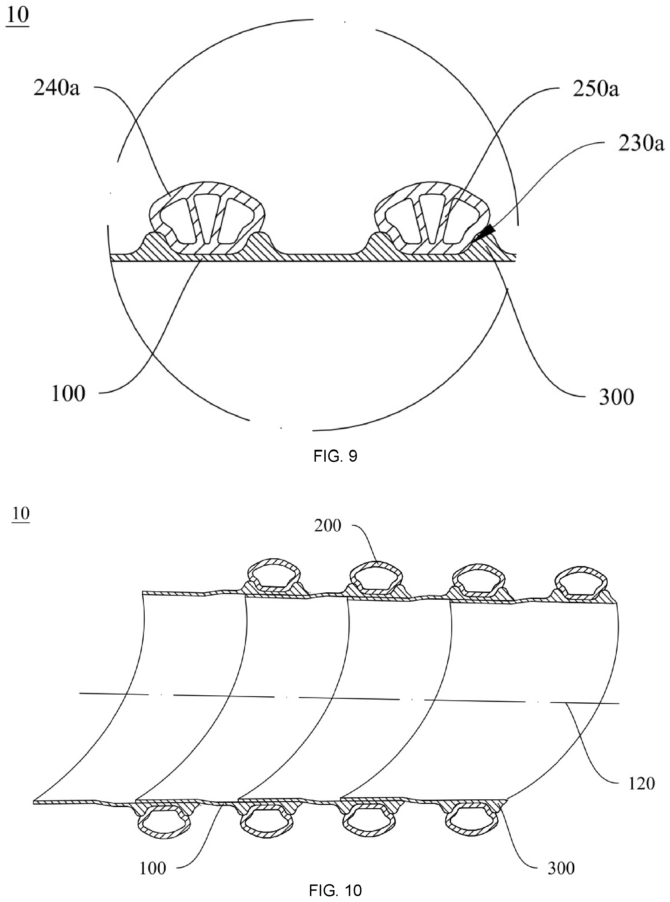

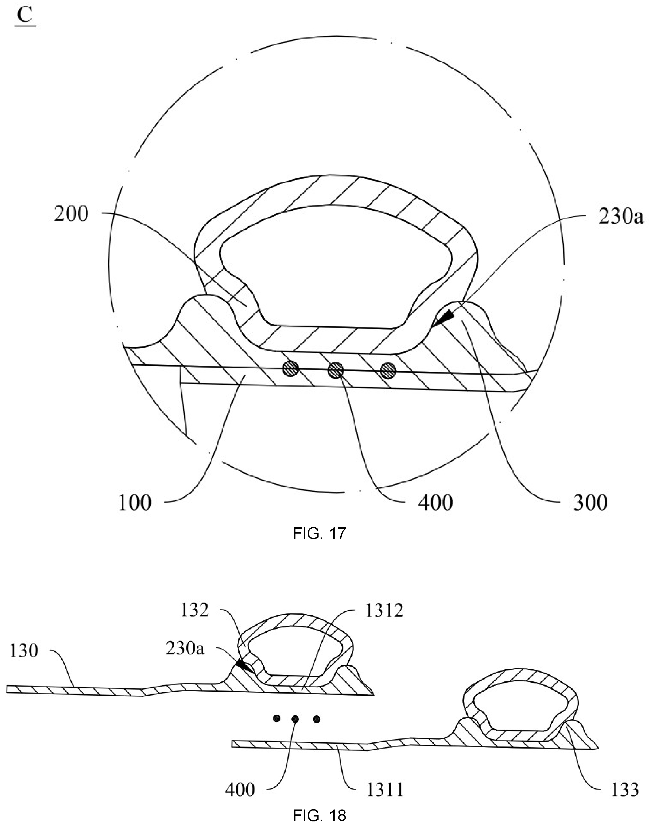

Regarding the specific structure of the gas guide tube 100, with reference to FIG. 10, in some embodiments, the gas guide tube 100 may be configured to be formed by spirally winding a strip 130. In different embodiments, there may be one or more strips 130. An embodiment in which the gas guide tube 100 is formed by winding only one strip 130 is described below as an example. Specifically, the strip 130 may include a strip body 131 and a lug 133. The strip body 131 has an inner overlapping portion 1311 on one side and an outer overlapping portion 1312 on another side along a width direction of the strip body. The width direction Y of the strip body 131 itself is defined as follows: along the direction perpendicular to the winding direction of the strip body 131, the relative direction between the two sides of the strip body 131 spirally wound around the outer peripheral wall surface 110 of the gas guide tube 100 is the width direction Y of the strip body 131 itself. Based on the above arrangement, with reference to FIG. 10 and FIG. 18, the outer overlapping portion 1312 and the inner overlapping portion 1311 of the strip body 131 in a spiral shape may be arranged in an overlapping manner, with the outer overlapping portion 1312 being located on the side of the inner overlapping portion 1311 away from the lumen of the gas guide tube 100. It will be understood that, along the spiral winding direction of the strip body 131, the outer overlapping portion 1312 of each turn of the wound strip body 131 overlaps the inner overlapping portion 1311 of the previous turn of the wound strip body 131, thereby forming a structure of the spiral strip body 131 with overlapping turns.

Based on the arrangement of the inner overlapping portion 1311 and the outer overlapping portion 1312 in the above embodiments, and in conjunction with the arrangement of the heating wire 400, with reference to FIG. 18, in some embodiments, the heating wire 400 is located between the inner overlapping portion 1311 and the outer overlapping portion 1312 along the radial direction of the gas guide tube 100. It will be understood that when the inner overlapping portion 1311 and the outer overlapping portion 1312 of the first support rib 200a form a self-overlapping wound structure, the heating wire 400 may be located between the inner overlapping portion 1311 and the outer overlapping portion 1312 of two adjacent turns. The above arrangement allows the heating wire 400 to be wound simultaneously while winding the first support rib 200a, thereby fixing both the first support rib 200a and the heating wire 400 at the same time, reducing the manufacturing time, and making the connection of the heating wire 400 more secure.

In some embodiments, with reference to FIG. 10, the support rib 200 is spirally wound around the gas guide tube 100 with the axis 120 of the gas guide tube 100 as the center axis. The helix angle and pitch of the support rib 200 may be determined according to the actual situations. It will be understood that a helix may be a left-hand helix or a right-hand helix.

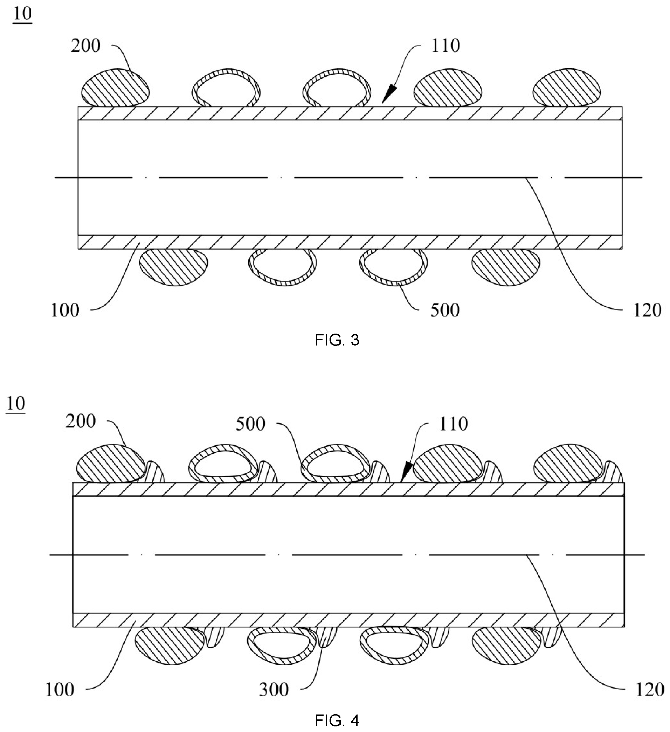

With reference to FIG. 4, a limiting protrusion 300 is provided on the outer peripheral wall surface 110 of the gas guide tube 100. The limiting protrusion 300 abuts against a spiral side edge 230a of the support rib 200 along the direction of the axis 120 of the gas guide tube 100. It should be noted that the spiral side edge 230a may be a helix on the outer periphery of the gas guide tube 100 that abuts against the spirally arranged support rib 200, i.e., the side edge that extends along the spiral winding direction of the first support rib 200a and defines the inner cavity of the first support rib 200a. It will be understood that the limiting protrusion 300 may be provided as a single protrusion or as a plurality of protrusions. Adapting to the functional requirements of limiting, in different embodiments, the limiting protrusion 300 may extend or be arranged in different forms. For example, the limiting protrusion 300 may be of a dot-shaped protruding structure, and a plurality of dot-shaped limiting protrusions 300 may be distributed on the outer peripheral wall surface 110. The limiting protrusion 300 may alternatively be strip-shaped. The strip-shaped limiting protrusion 300 may be annularly wound around the outer peripheral wall surface 110 of the gas guide tube 100, or may be spirally wound around the outer peripheral wall surface 110 of the gas guide tube 100. In some embodiments of the present disclosure, an example in which two limiting protrusions 300 are provided, both of which are spirally disposed around the outer peripheral wall surface 110 of the gas guide tube 100, is used for illustration.

In some embodiments, one lug 133 is provided, such that the gas guide tube 100 includes one limiting protrusion 300. That is, a single lug 133 forms the limiting protrusion 300 after being spirally wound. Along the axial direction of the gas guide tube 100, one side of the limiting protrusion 300 abuts against the spiral side edge 230a on one side of the first support rib 200a, while another side of the limiting protrusion 300 abuts against the spiral side edge 230a on another side of the first support rib 200a. According to this solution, the lug 133 is made of less material, thereby saving the material used for manufacturing.

In some embodiments, the number of lugs 133 is two, such that the gas guide tube 100 includes two limiting protrusions 300. That is, two lugs 133 form two limiting protrusions 300 after being spirally wound. The first support rib 200a is located between the two limiting protrusions 300. That is, the two limiting protrusions 300 can clamp the first support rib 200a. According to this solution, by providing two limiting protrusions 300, the stability of the connection between the gas guide tube 100 and the first support rib 200a is further improved.

The arrangement of the heating wire 400 may correspond to that of the limiting protrusions 300 (lugs 133) in the above embodiments. Specifically, corresponding to the above arrangement that the first support rib 200a is located between two limiting protrusions 300, in some embodiments, two limiting protrusions 300 may be provided on the outer peripheral wall surface 110 of the gas guide tube 100. The two limiting protrusions 300 may abut against the two opposite spiral side edges 230a of the first support rib 200a along the direction of the axis 120 of the gas guide tube 100, respectively. Based on the above arrangement, in some embodiments, the heating wire 400 may be located between the two limiting protrusions 300 along the axial direction of the gas guide tube 100. It will be understood that the two limiting protrusions 300 respectively abutting against the two sides of the first support rib 200a can limit and fix the first support rib 200a, while arranging the heating wire 400 between the two limiting protrusions 300 can make the connection/pressing action of the first support rib 200a on the heating wire 400 stronger, and also make the heat preservation and insulation effect provided by the first support rib 200a better. Furthermore, in some embodiments, the two limiting protrusions 300 may be spirally wound around the gas guide tube 100 with the axis 120 of the gas guide tube 100 as the center axis.

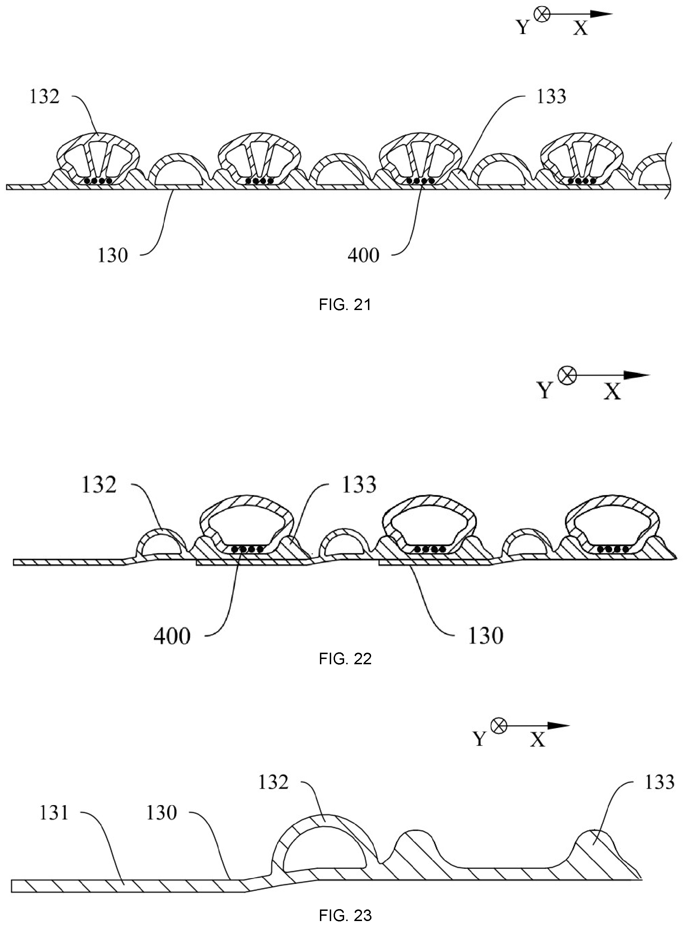

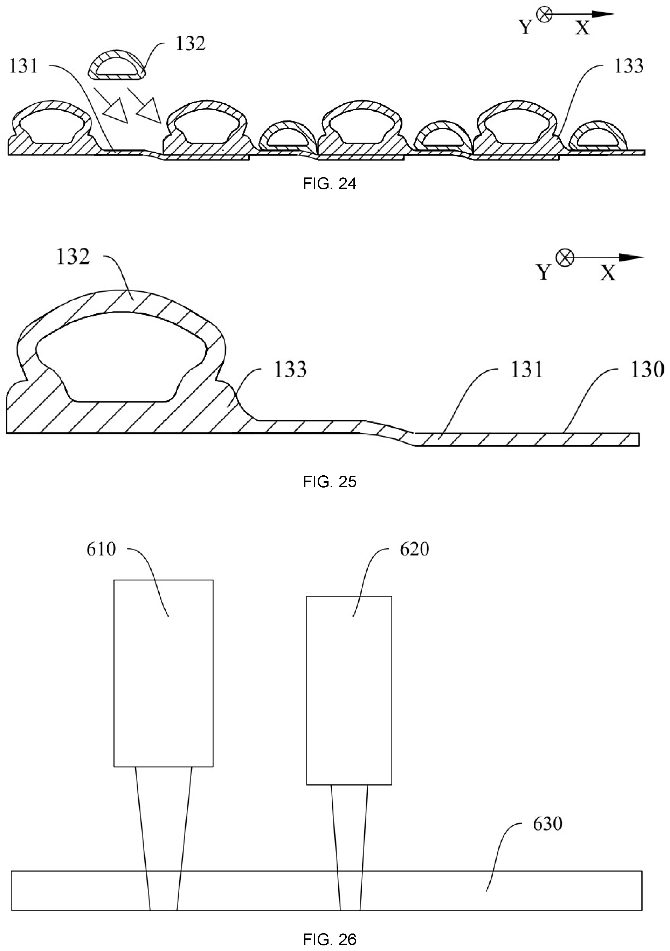

Regarding the winding form of the first support rib 200a or the second support rib 200b, with reference to FIG. 23 or FIG. 25, in some embodiments, the first support rib 200a may be integrated with the gas guide tube 100, and the gas guide tube 100 may be configured to be formed by spirally winding a strip 130. The strip 130 includes a strip body 131 and a support strip 132. The support strip 132 is connected to a side of the strip body 131, and defines, together with the strip body 131, an inner cavity extending along the length direction X of the strip 130. It will be understood that the support strip 132 may extend along the length direction X of the strip body 131, and the cross-section of the support strip 132 may be semicircular, arched, open rectangular, or the like, such that the wall surface of the strip body 131 on the side away from the inner cavity of the breathing tube 10 may define, together with the support strip 132, the inner cavity extending along the length direction X of the strip 130. Similar to the principle of forming the limiting protrusion 300 in the above embodiments, the support strip 132 may form the first support rib 200a after being spirally wound. That is, when the strip body 131 is spirally wound, the support strip 132 may correspondingly form a spiral shape to form the first support rib 200a.

Similarly, for the second support rib 200b, with reference to FIG. 21 or FIG. 22, in some embodiments, the second support rib 200b may be integrated with the gas guide tube 100. The gas guide tube 100 may be configured to be formed by spirally winding a strip 130. The strip 130 includes a strip body 131 and a support strip 132. The support strip 132 may be connected to a side of the strip body 131, and defines, together with the strip body 131, an inner cavity extending along the length direction X of the strip 130. The support strip 132 may form the second support rib 200b after being spirally wound. It will be understood that the manner of winding the second support rib 200b in this embodiment is similar to that in the previous embodiment. Therefore, the definitions relating to the second support rib 200b in this embodiment may refer to those of the first support rib 200a in the above embodiments, and such embodiments may be implemented simultaneously.

The above embodiments are based on the arrangement in which the first support rib 200a/second support rib 200b is formed by winding the support strip 132, and defines, together with the strip body 131, the inner cavity. Further, the limiting protrusion 300 may have different positional relationships with the first support rib 200a/second support rib 200b. Specifically, in an embodiment of one positional relationship, the first support rib 200a may be integrated with the gas guide tube 100. The gas guide tube 100 may be configured to be formed by spirally winding a strip 130. The strip 130 may include a strip body 131, a support strip 132, and a lug 133. The support strip 132 is connected to a side of the strip body 131, and defines, together with the strip body 131, an inner cavity extending along the length direction X of the strip 130. The support strip 132 forms the first support rib 200a after being spirally wound. The lug 133 may be connected to the strip body 131 and located on the same side as the support strip 132. Based on the above arrangement, the lug 133 may extend along the length direction X of the strip body 131 and be spaced apart from the support strip 132 along the width direction Y of the strip body 131. The lug 133 forms the limiting protrusion 300 after being spirally wound. It will be understood that this arrangement combines the arrangements of the lug 133 and the support strip 132 from the above embodiments. In particular, the first support rib 200a is located between the limiting protrusion 300 and the second support rib 200b. It will be understood that, along the axial direction of the gas guide tube 100, one side of the first support rib 200a faces the limiting protrusion 300, and the opposite side faces the second support rib 200b. Both sides of the first support rib 200a may abut against or be spaced apart from the limiting protrusion 300 or the second support rib 200b. The above arrangement enables both the limiting protrusion 300 and the second support rib 200b to limit the first support rib 200a, and the limiting protrusion 300 is configured to define the pitch formed by the first support rib 200a and the second support rib 200b. The second support rib 200b can also enhance supporting strength and thermal insulation. Moreover, using the second support rib 200b for limiting on one side of the first support rib 200a can save material, reduce manufacturing costs, and lower weight.

With reference to FIG. 19 and FIG. 20, in an embodiment of another positional relationship, the strip 130 may further include two lugs 133. The two lugs 133 may be connected to the strip body 131. The two lugs 133 extend along the length direction X of the strip body 131 and are spaced apart from each other along the width direction Y of the strip body 131. The two lugs 133 form two limiting protrusions 300 after being spirally wound. In particular, the first support rib 200a is located between the two limiting protrusions 300. It will be understood that, along the axial direction of the gas guide tube 100, the two opposite sides of the first support rib 200a each face one of the limiting protrusions 300. Further, in some embodiments, the strip 130 may further include four lugs 133. The four lugs 133 form four limiting protrusions 300 after being spirally wound. The first support rib 200a is located between two of the limiting protrusions 300, and the second support rib 200b is located between the other two limiting protrusions 300. That is to say, the first support rib 200a and the second support rib 200b may each be located between two limiting protrusions 300.

In this solution, the strip body 131 and the lugs 133 may be formed separately and then joined and wound, or may be integrally formed. Thus, high manufacturing efficiency is achieved. Specifically, this is also based on the arrangement of the inner overlapping portion 1311 and the outer overlapping portion 1312 in the above embodiments. With reference to FIG. 24 or FIG. 25, in some embodiments, the lug 133 may be connected to the outer overlapping portion 1312. The above arrangement allows the structure of the lug 133 to avoid interference from the spirally wound strip body 131. In some embodiments, the lug 133 may extend along the length direction X of the strip body 131, and the lug 133 in the spiral shape is the limiting protrusion 300. It will be understood that the extension of the strip body 131 along its length direction X corresponds to the spiral winding direction of the strip body 131. That is, when the strip body 131 is spirally wound, the lug 133 extending along the length direction X of the strip body 131 may correspondingly form a spiral shape so as to form the limiting protrusion 300.

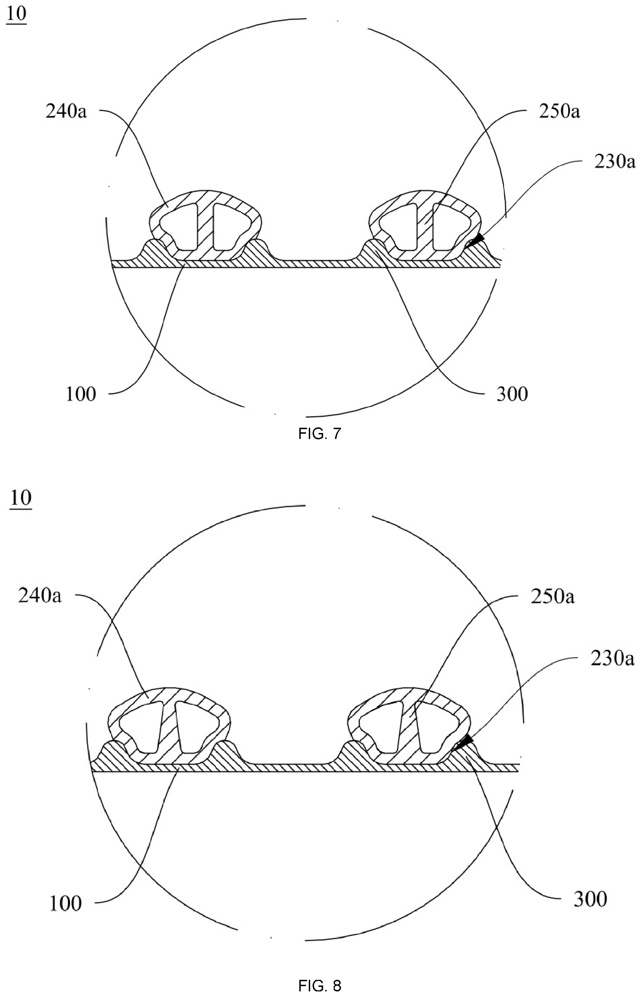

With reference to FIG. 7 to FIG. 9, in some embodiments, the first support rib 200a may include a ribbed tube 240a and a rib strip 250a. The ribbed tube 240a is connected to the outer peripheral wall surface 110 of the gas guide tube 100, and the rib strip 250a is disposed within the ribbed tube 240a. Specifically, in some embodiments, the rib strip 250a may extend along the direction of the axis 120 of the gas guide tube 100. In some other embodiments, the rib strip 250a may extend along the direction perpendicular to the axis 120 of the gas guide tube 100. In some embodiments of the present disclosure, an example in which the rib strip 250a extends along the direction of the axis 120 of the gas guide tube 100 is used for illustration. In some embodiments, the second support rib 200b may alternatively include a ribbed tube 240a and a rib strip 250a, and have a similar arrangement to the first support rib 200a in the above embodiments. For ease of description, an embodiment in which only the first support rib 200a includes the ribbed tube 240a and the rib strip 250a is described below as an example. Different embodiments may be combined with one another among different technical solutions.

The side of the rib strip 250a close to the gas guide tube 100 is connected to the inner wall surface of the side of the ribbed tube 240a close to the gas guide tube 100, and the side of the rib strip 250a away from the gas guide tube 100 is connected to the inner wall surface of the side of the ribbed tube 240a away from the gas guide tube 100. That is, the rib strip 250a divides the ribbed tube 240a into two independent spiral spaces. According to this solution, by arranging the rib strip 250a within the ribbed tube 240a, the structural strength of the ribbed tube 240a can be effectively improved, the stability of the support provided by the ribbed tube 240a to the gas guide tube 100 can be enhanced, and collapse of the ribbed tube 240a can be avoided.

The rib strip 250a may extend in different directions. With reference to FIG. 7 to FIG. 9, in some embodiments, the rib strip 250a may extend in a direction perpendicular to the axis 120 of the gas guide tube 100 while maintaining a constant distance from the axis 120 of the gas guide tube 100 at all points (in the embodiment corresponding to FIG. 7 to FIG. 9, this direction is perpendicular to the plane of the drawing), and may be connected to the wall surfaces on opposite sides of the gas guide tube 100 in this direction. In some other embodiments, the rib strip 250a may extend along the direction of the axis 120 of the gas guide tube 100 and be connected to the wall surfaces on opposite sides of the gas guide tube 100 in this direction.

In some embodiments, the rib strip 250a may be disposed spirally with the axis 120 of the gas guide tube 100 as the center axis. It will be understood that, based on the above arrangement, the ribbed tube 240a may independently define, or define, together with the wall surface of the side of the gas guide tube 100 close to the ribbed tube 240a, the inner cavity of the first support rib 200a. The ribbed tube 240a may be disposed in the inner cavity. The ribbed tube 240a may be disposed spirally with the axis 120 of the gas guide tube 100 as the center axis, thereby forming the spirally wound first support rib 200a. To conform to the spirally wound ribbed tube 240a and to provide the first support rib 200a with better supporting strength, the rib strip 250a may also be disposed spirally.

In some embodiments, with reference to FIG. 8, the thickness of the rib strip 250a decreases gradually along a direction away from the gas guide tube 100. In some other embodiments, with reference to FIG. 7, the thickness of the rib strip 250a is uniform along the direction away from the gas guide tube 100. In other embodiments, the thickness of the rib strip 250a increases gradually along the direction away from the gas guide tube 100. The specific arrangement of the rib strip 250a may be determined according to the actual situations.

With reference to FIG. 9, in some embodiments, the first support rib 200a may include a plurality of rib strips 250a. Specifically, two, three, four, or more rib strips 250a may be provided. In some embodiments of the present disclosure, two rib strips 250a are taken as an example for illustration. The rib strips 250a are arranged at intervals along the radial direction of the ribbed tube 240a. Along the direction away from the gas guide tube 100, spacings between the ribbed tubes 240a increase gradually, which can adapt to the curved structure of the ribbed tube 240a and ensure the stability of the support provided by the rib strips 250a to the ribbed tube 240a. In some other embodiments, the first support rib 200a may include a plurality of rib strips 250a. The rib strips 250a are arranged at intervals along the radial direction of the ribbed tube 240a, and along the direction away from the gas guide tube 100, the spacings between the ribbed tubes 240a are uniform. This depends on the actual situations.

In some embodiments, the breathing tube 10 meets at least one of the following conditions a) to k):
a) A wall thickness d₁ of the gas guide tube 100 meets: 0.13 mm≤d₁≤0.23 mm. With reference to FIG. 6, exemplarily, d₁ may be 0.13 mm, 0.16 mm, 0.18 mm, 0.21 mm, or 0.23 mm. It will be understood that the wall thickness of the gas guide tube 100 may be uniform.
b) A wall thickness d₂ of the hollow segment 500 meets: 0.1 mm≤d₂≤0.5 mm. With reference to FIG. 6, exemplarily, d₂ may be 0.1 mm, 0.17 mm, 0.3 mm, 0.45 mm, or 0.5 mm. It will be understood that the wall thickness of the first support rib 200a may be uniform or non-uniform.
c) An inner diameter D₁ of the gas guide tube 100 meets: 8 mm≤D₁≤28 mm. With reference to FIG. 5, exemplarily, D₁ may be 8 mm, 11 mm, 16 mm, 20 mm, 27 mm, or 28 mm. It will be understood that the inner diameter of the gas guide tube 100 may be identical or different at different positions.
d) Along the radial direction of the gas guide tube 100, a height dimension L₁ of the hollow segment 500 meets: 2.2 mm≤L₁≤2.8 mm. With reference to FIG. 5, exemplarily, L₁ may be 2.2 mm, 2.4 mm, 2.5 mm, 2.7 mm, or 2.8 mm.
e) Along the axial direction of the gas guide tube 100, a width dimension L₂ of the hollow segment 500 meets: 2 mm≤L₂≤6 mm. With reference to FIG. 5, exemplarily, L₂ may be 2 mm, 2.4 mm, 4 mm, 4.7 mm, 5 mm, or 6 mm.
   It should be noted that the above-mentioned height dimension L₁ and width dimension L₂ are the cross-sectional dimensions of a single turn of the wound first support rib 200a. That is, it can be understood that the cross-section of the hollow segment 500 along the direction perpendicular to the axis 120 of the gas guide tube 100 includes a plurality of cross-sectional units arranged along the direction of the axis 120 of the gas guide tube 100, and the plurality of cross-sectional units are distributed on the outer peripheral walls on both sides of the gas guide tube 100. In this case, the height dimension L₁ and the width dimension L₂ are both the dimensions of a single cross-sectional unit distributed on the outer peripheral wall of the same side of the gas guide tube 100. The following describes an embodiment in which the strip 130 includes a strip body 131 and a support strip 132, the support strip 132 is connected to one side of the strip body 131 and defines, together with the strip body 131, an inner cavity extending along the length direction X of the strip 130, and the support strip 132 forms the hollow segment 500 after being spirally wound. In this case, the height dimension L₁ and the width dimension L₂ are the height dimension L₁ and the width dimension L₂ of a single support strip 132.
f) The hollow segment 500 is arranged spirally with the axis 120 of the gas guide tube 100 as the center axis, and the lead S of the hollow segment meets: 2 mm≤S≤8 mm. Exemplarily, S may be 2 mm, 4 mm, 5 mm, 7 mm, or 8 mm.
g) The first support rib 200a is spirally wound around the gas guide tube 100 with the axis 120 of the gas guide tube 100 as the center axis. A limiting protrusion 300 is provided on the outer peripheral wall surface 110 of the gas guide tube 100. The limiting protrusion 300 abuts against the spiral side edge 230a of the first support rib 200a along the direction of the axis 120 of the gas guide tube 100. With reference to FIG. 5 and FIG. 6, along the radial direction of the gas guide tube 100, the height dimension L₃ of the limiting protrusion 300 meets: 0.3 mm≤L₃≤1.3 mm. Exemplarily, L₃ may be 0.3 mm, 0.5 mm, 0.8 mm, 0.9 mm, 1.2 mm, or 1.3 mm.
h) The first support rib 200a is spirally wound around the gas guide tube 100 with the axis 120 of the gas guide tube 100 as the center axis. A limiting protrusion 300 is provided on the outer peripheral wall surface 110 of the gas guide tube 100. The limiting protrusion 300 abuts against the spiral side edge 230a of the first support rib 200a along the direction of the axis 120 of the gas guide tube 100. With reference to FIG. 5, along the axial direction of the gas guide tube 100, the width dimension L₄ of the limiting protrusion 300 meets: 0.5 mm≤ L₄≤1.5 mm. Exemplarily, L₄ may be 0.3 mm, 0.5 mm, 0.8 mm, 0.9 mm, 1.2 mm, or 1.3 mm.
i) The first support rib 200a is arranged spirally with the axis 120 of the gas guide tube 100 as the center axis, and the lead of the first support rib 200a is greater than the width dimension of the first support rib 200a along the axial direction of the gas guide tube 100. The lead of the first support rib 200a refers to a distance by which a tool moves along the direction of the axis 120 within a range of one revolution of the first support rib 200a around the gas guide tube 100.
j) A second support rib 200b is further wound around the gas guide tube 100. With reference to FIG. 20, along the radial direction of the gas guide tube 100, the height dimension L₅ of the second support rib 200b meets: 1.3 mm≤L₅≤2.3 mm. Exemplarily, L₅ may be 1.5 mm, 1.8 mm, 2 mm, or 2.2 mm.
k) A second support rib 200b is further wound around the gas guide tube 100. With reference to FIG. 20, along the axial direction of the gas guide tube 100, the width dimension L₆ of the second support rib 200b meets: 1.5 mm≤L₆≤2.5 mm. Exemplarily, L₆ may be 1.8 mm, 2 mm, 2.2 mm, or 2.4 mm.

In some embodiments, the gas guide tube 100 may be configured to be formed by spirally winding a strip 130. It will be understood that the strip 130 may be formed through an extrusion process.

In some embodiments, the gas guide tube 100 is made of a transparent material or a translucent material. By forming the gas guide tube 100 from a transparent material or a translucent material, this solution facilitates direct observation of gas flow within the tube, avoids situations where a patient's abnormal breathing occurs without timely detection, and ensures medical safety. It should be noted that the gas guide tube 100 should have a certain degree of flexibility, allowing the support rib 200 to be compressed or bent.

In some embodiments, the support rib 200 is made of a transparent material or a translucent material. By forming the support rib 200 from a transparent material, this solution facilitates observation of the support of the support rib 200. That is, when the support rib 200 is damaged (e.g., due to impact) and cracks, relevant personnel can promptly detect this abnormal condition and take measures to ensure patient safety. It should be noted that the support rib 200 should maintain bubble-point integrity without significant collapse or depression under natural conditions as well as when bent or compressed at a minimum radius. Specifically, the material of the support rib 200 may be thermoplastic elastomer (TPE), polyolefin elastomer (POE), polypropylene (PP), thermoplastic polyurethane (TPU), polyamide (PA) 12, or the like.

With reference to FIG. 3, an embodiment of a second aspect of the present disclosure provides a breathing tube 10. The breathing tube 10 includes a gas guide tube 100 and a first support rib 200a. The gas guide tube 100 is configured to convey a gas. The first support rib 200a is connected to an outer peripheral wall surface 110 of the gas guide tube 100. The first support rib 200a is disposed around an axis 120 of the outer peripheral wall surface 110. The first support rib 200a includes a hollow segment 500. In particular, the hollow segment 500 and the outer peripheral wall surface 110 of the gas guide tube 100 together define a cavity. It will be understood that the breathing tube 10 is similar to the breathing tube 10 of the embodiments of the first aspect described above, with the difference being that in the breathing tube 10 of the embodiments of the second aspect of the present disclosure, the hollow segment 500 and the outer peripheral wall surface 110 of the gas guide tube 100 together define the cavity. The above arrangement allows the gas guide tube 100 and the hollow segment 500 to be integrated, so that in some embodiments, the gas guide tube 100 and the hollow segment 500 can be simultaneously formed through an extrusion process, thereby improving the manufacturing efficiency.

An embodiment of a third aspect of the present disclosure provides a medical device. The medical device includes the breathing tube 10 in any of the above embodiments. It will be understood that the medical device may be a ventilator. Compared with existing breathing tubes 10, the breathing tube 10 in the medical device of this solution has lower weight, can ensure the stability of the connection between the breathing tube 10 and the patient, save the material, reduce the production cost, and can further effectively suppress heat exchange between the flowing medium within the tube and the external environment, thereby achieving the heat preservation and insulation effect.

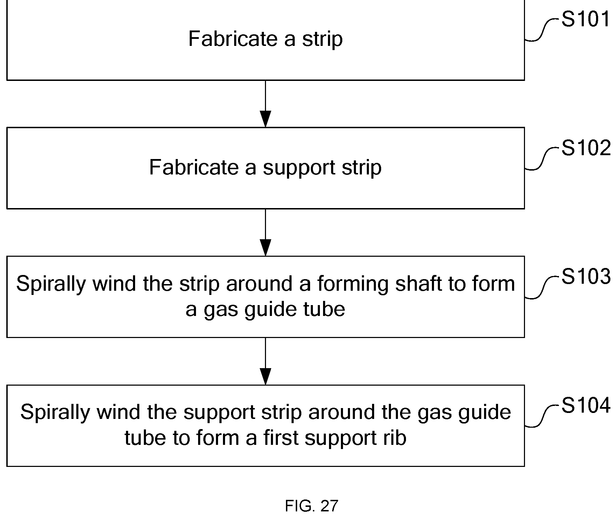

An embodiment in a fourth aspect of the present disclosure provides a manufacturing method, which is used for manufacturing the breathing tube 10 in the above embodiments. With reference to FIG. 27, the manufacturing method includes the following steps.

In step S101, the strip 130 is fabricated. Specifically, the strip 130 may be formed through extrusion molding using an extruder. It will be understood that the strip 130 may be made of a transparent material or a translucent material. By forming the strip 130 from a transparent material or a translucent material, this solution facilitates direct observation of gas flow within the tube, avoids situations where a patient's abnormal breathing occurs without timely detection, and ensures medical safety. It should be noted that the strip 130 should have a certain degree of flexibility, allowing the support rib 200 to be compressed or bent.

In step S102, the support strip 132 is fabricated. The support strip 132 is formed through extrusion molding using an extruder. It will be understood that the support strip 132 may be made of a transparent material or a translucent material. By forming the support strip 132 from a transparent material, this solution facilitates observation of the support of the support strip 132. That is, when the support strip 132 is damaged (e.g., due to impact) and cracks, relevant personnel can promptly detect this abnormal condition and take measures to ensure patient safety. It should be noted that the support strip 132 should maintain shape integrity without significant collapse or depression under natural conditions as well as when bent or compressed at a minimum radius. Specifically, the material of the support strip 132 may be TPE, POE, PP, TPU, PA12, or the like. This depends on the actual situations.

In step S103, the strip 130 is spirally wound around a forming shaft 630 to form the gas guide tube 100. Specifically, the forming shaft 630 may drive the strip 130 to rotate such that the strip 130 is wound around the forming shaft. It should be noted that the forming shaft 630 may be connected to an external power source to provide power for conveying and winding the strip 130 and for rotating the forming shaft 630. Separate power sources may be provided for the strip 130 and the forming shaft 630. Alternatively, the strip 130 and the forming shaft 630 may share a power source. This depends on the actual situations.

The strip 130 can be stacked and lapped on the forming shaft 630 at a set pitch to form the gas guide tube 100. Specifically, along the spiral winding direction of the strip body 131, the outer overlapping portion 1312 of each turn of the wound strip body 131 overlaps the inner overlapping portion 1311 of the previous turn of the wound strip body 131, thereby forming a structure of the spiral strip body 131 with overlapping turns, i.e., forming the gas guide tube 100.

In step S104, the support strip 132 is spirally wound around the gas guide tube 100 to form the first support rib 200a and the second support rib 200b. Specifically, the forming shaft 630 may drive the support strip 132 to rotate such that the support strip 132 is wound around the gas guide tube 100, thereby forming the first support rib 200a and the second support rib 200b. The structures of the first support rib 200a and the second support rib 200b may be the same or different. In some embodiments of the present disclosure, an example in which the structures of the first support rib 200a and the second support rib 200b are different is used for illustration.

It should be noted that, in some embodiments, the gas guide tube 100 may be formed first (i.e., the strip 130 is wound first), and then the first support rib 200a and the second support rib 200b are formed (i.e., the support strip 132 is wound around the gas guide tube 100), thereby forming the breathing tube 10. In some other embodiments, the strip 130 and the support strip 132 may be fabricated simultaneously, and the support strip 132 is wound around the gas guide tube 100 while the strip 130 is wound around the forming shaft 630. In some embodiments of the present disclosure, the latter approach is taken as an example, i.e., the gas guide tube 100, the first support rib 200a, and the second support rib 200b are formed simultaneously. This approach provides higher forming efficiency and ensures the manufacturing efficiency of the breathing tube 10.

In some embodiments, the step of fabricating the strip 130 may specifically include the following:
The strip 130 is formed through an extrusion molding process using a first extrusion unit 610. First, the raw material for the strip 130 may be pretreated, including processes such as drying, desiccating, and mixing of the granular raw material, to ensure the raw material quality and the manufacturing effect. Second, the pretreated raw material is placed in the first extrusion unit 610 for melting. The first extrusion unit 610 gradually melts the raw material into a molten plastic paste through heating, stirring, pressing, and the like. Finally, the molten plastic paste is extruded from the die of the first extrusion unit 610 to form the desired cross-sectional shape.

The support strip 132 is formed through an extrusion molding process using a second extrusion unit 620. The extrusion process of the support strip 132 is similar to that of the strip 130 and will not be repeated here. It will be understood that the second extrusion unit 620 may be the same as the first extrusion unit 610.

Further, in conjunction with the arrangement of the heating wire 400, in some embodiments, the step of fabricating the strip 130 may specifically include the following:

The heating wire 400 and the strip 130 are integrally formed through extrusion molding, where the heating wire 400 is located within the strip 130.

Alternatively,
the step of fabricating the support strip 132 may specifically include integrally forming the heating wire 400 and the support strip 132 through extrusion molding, where the heating wire 400 is located within the support strip 132.

Alternatively,
before the step of spirally winding the support strip 132 around the gas guide tube 100 to form the first support rib 200a, the method includes spirally winding the heating wire 400 around the gas guide tube 100, spirally winding the support strip 132 around the gas guide tube 100, and covering the heating wire 400 with the support strip 132.

It will be understood that in the above steps, integrally forming the heating wire 400 and the strip 130/support strip 132 through extrusion molding means that the heating wire 400 first passes through the strip 130/support strip 132 so that the heating wire 400 and the strip 130/support strip 132 can be simultaneously manufactured by co-extrusion molding. This approach provides higher forming efficiency and a more stable connection of the heating wire 400. Furthermore, spirally winding the heating wire 400 around the gas guide tube 100, spirally winding the support strip 132 around the gas guide tube 100, and covering the heating wire 400 with the support strip 132 mean that after the heating wire 400 is wound around the outer peripheral wall of the gas guide tube 100, the support strip 132 is then spirally wound around the gas guide tube 100 and covers the heating wire 400, thereby fixing and holding down the heating wire 400. This approach allows the step of winding the heating wire 400 and the step of integrally manufacturing through co-extrusion molding to be independent of each other, thereby helping to reduce the damage rate of the heating wire 400 and improve the manufacturing quality of the co-extrusion molding.

In some embodiments, the step of spirally winding the strip 130 around the forming shaft 630 to form the gas guide tube 100 may specifically include the following:

The first extrusion unit 610 is disposed beside the forming shaft 630. Specifically, the first extrusion unit 610 may be located on the upper side of the forming shaft 630 in the circumferential direction. The specific arrangement may be determined according to the actual situations.

During the process of forming the strip 130 by the first extrusion unit 610, the formed strip 130 is wound around the forming shaft 630. That is, the gas guide tube 100 may be formed simultaneously with the formation of the strip 130. The freshly extruded strip 130 has high plasticity, which facilitates the winding and forming of the gas guide tube 100. Thus, the manufacturing efficiency of the gas guide tube 100 can be effectively improved, and the manufacturing quality of the gas guide tube 100 can be ensured.

In some embodiments, the step of spirally winding the support strip 132 around the gas guide tube 100 to form the first support rib 200a and the second support rib 200b may specifically include the following:

The second extrusion unit 620 is disposed beside the forming shaft 630. It will be understood that the second extrusion unit 620 may be spaced apart from the first extrusion unit 610, and the second extrusion unit 620 may be disposed at a portion other than the ends of the forming shaft 630. During the process of forming the support strip 132 by the second extrusion unit 620, the formed support strip 132 is wound around the formed gas guide tube 100. That is, the first support rib 200a and the second support rib 200b may be formed simultaneously with the formation of the support strip 132. The freshly extruded support strip 132 has high plasticity, which facilitates the winding and forming of the support rib 200. Thus, the manufacturing efficiency of the first support rib 200a and the second support rib 200b can be effectively improved, and the manufacturing quality can be ensured.

In some embodiments, the step of spirally winding the strip 130 around the forming shaft 630 to form the gas guide tube 100 may further include the following:
A plurality of flexible spring shafts are disposed along the circumferential direction of the forming shaft 630. The specific number of flexible spring shafts may be determined according to the actual situations. The strip 130 is spirally wound around the flexible spring shafts, and the flexible spring shafts are driven by a driver to move along the axial direction of the forming shaft 630 and to rotate along the circumferential direction of the forming shaft 630, thereby driving the strip 130 to move spirally. In this way, the strip 130 is spirally wound around the forming shaft 630. In this solution, by providing the driver, it is convenient to produce the gas guide tubes 100 with different specifications and dimensions according to actual requirements, and the forming quality of the gas guide tube 100 can be ensured.

In some embodiments, the above-described manner of driving the flexible spring shafts to move so as to drive the strip 130 may be specifically expressed as: driving the flexible spring shafts to move spirally along the axial direction of the forming shaft 630, while integrally forming the strip 130 through extrusion molding, and during the formation of the strip 130, winding the formed strip 130 around the forming shaft 630 and covering the flexible spring shafts. The above actions allow the strip 130 covering the flexible spring shafts to move in the opposite direction as the flexible spring shafts move spirally, thereby enabling the flexible spring shafts to automatically spirally wind the strip 130. Thus, the manufacturing efficiency is improved. After the winding of the strip 130 is completed, the flexible spring shafts can be cut and withdrawn. In some embodiments, while driving the flexible spring shafts, the support strip 132 may be integrally formed through extrusion molding, so that the movement of the flexible spring shafts can simultaneously drive the strip 130 and the support strip 132 to be spirally wound. Thus, the manufacturing efficiency is further improved. Furthermore, to facilitate the spiral movement of the flexible spring shafts along the axial direction of the forming shaft 630, a spiral groove may be provided on the surface of the shaft body of the forming shaft 630, so that the flexible spring shafts can extend into the spiral groove and move along the extension direction thereof.

In some embodiments, the step of fabricating the support strip 132 may specifically include the following:
Two support strips 132 are fabricated. It will be understood that two support strips 132 may be extruded simultaneously using the second extrusion unit 620, or one of the support strips 132 may be extruded using the second extrusion unit 620 while the other support strip 132 is extruded using an additional extrusion unit. In some embodiments of the present disclosure, the former approach (using the second extrusion unit 620 to extrude two support strips 132 simultaneously) is taken as an example for illustration.

In some embodiments, the step of spirally winding the support strip 132 around the gas guide tube 100 includes the following:
The two support strips 132 are spirally wound around the gas guide tube 100 to form a dual-spiral structure, thereby forming the first support rib 200a and the second support rib 200b. By forming the first support rib 200a and the second support rib 200b, this solution can further improve the stability of the support for the gas guide tube 100, prevent significant bending of the gas guide tube 100, and enhance the heat preservation and insulation performance of the gas guide tube 100.

The arrangement of the first extrusion unit 610, the second extrusion unit 620, and the forming shaft 630 in the above embodiments may refer to FIG. 26.

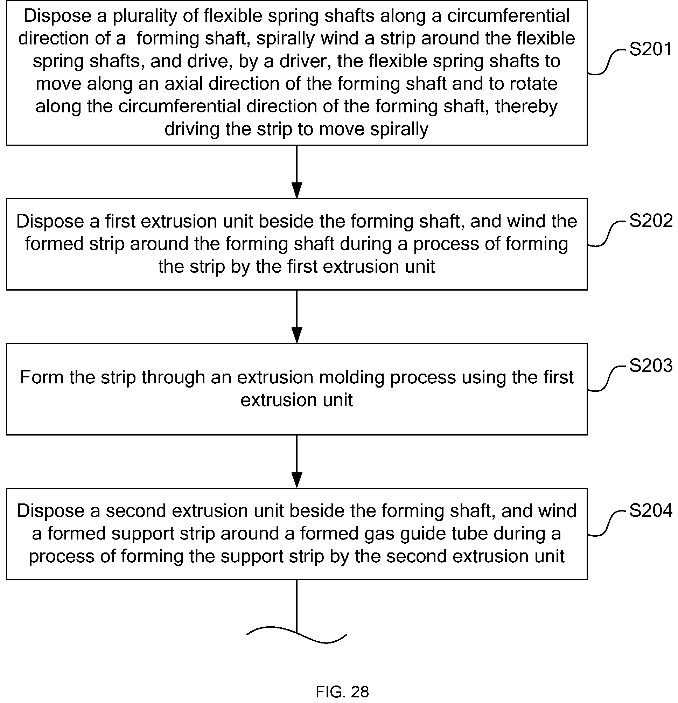

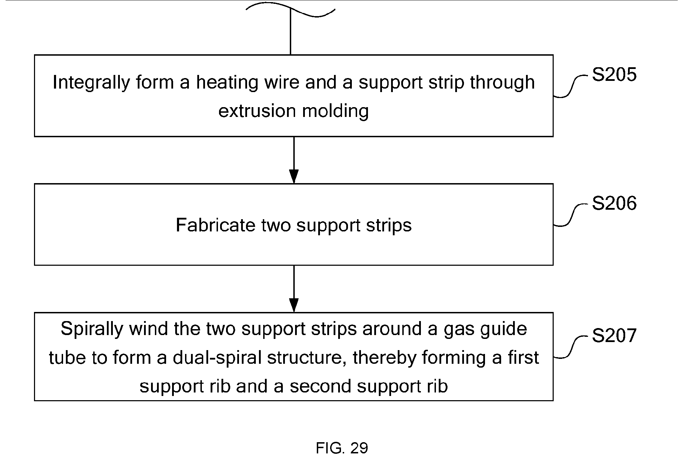

For ease of understanding, a manufacturing method corresponding to a complete embodiment of the present disclosure is described below. With reference to FIG. 28 and FIG. 29, schematic flowcharts of a manufacturing method according to another embodiment of the present disclosure are shown. The method includes, but is not limited to, the following steps:
In step S201, a plurality of flexible spring shafts are disposed along the circumferential direction of a forming shaft 630. The strip 130 is spirally wound around the flexible spring shafts, and the flexible spring shafts are driven by a driver to move along the axial direction of the forming shaft 630 and to rotate along the circumferential direction of the forming shaft 630, thereby driving the strip 130 to move spirally.

In step S202, a first extrusion unit 610 is disposed beside the forming shaft 630, and the formed strip 130 is wound around the forming shaft 630 during the process of forming the strip 130 by the first extrusion unit 610.

In step S203, the strip 130 is formed through an extrusion molding process using the first extrusion unit 610.

In step S204, a second extrusion unit 620 is disposed beside the forming shaft 630, and the formed support strip 132 is wound around the formed gas guide tube 100 during the process of forming the support strip 132 by the second extrusion unit 620.

In step S205, the heating wire 400 and the support strip 132 are integrally formed through extrusion molding, where the heating wire 400 is located within the support strip 132.

In step S206, two support strips 132 are fabricated, where at least one support strip 132 includes the hollow segment 500.

In step S207, the two support strips 132 are spirally wound around the gas guide tube 100 to form a dual-spiral structure, thereby forming the first support rib 200a and the second support rib 200b.

The foregoing are merely preferred embodiments of the present disclosure, and the scope of the present disclosure is not limited thereto. Any equivalent structure change made using the content of the specification of the present disclosure and the accompanying drawings under the application concept of the present disclosure, or direct/indirect application thereof in other related technical fields, shall fall within the protection scope of the present disclosure.

It should be noted that all the directional indications such as upper, lower, left, right, front, and rear in the embodiments of the present disclosure are merely used to explain relative position relationships or motion situations of the components in a specific gesture. If the specific gesture changes, the directional indication changes accordingly.

In addition, the terms such as "first", "second", or the like described in the embodiments of the present disclosure are used herein only for the purpose of description and are not intended to indicate or imply relative importance, or implicitly indicate the number of indicated technical features. Therefore, a feature limited by "first" or "second" may explicitly or implicitly include at least one such feature. In addition, the meaning of "and/or" used throughout the specification includes three parallel solutions. Taking "A and/or B" as an example, it includes a solution A, a solution B, and a solution of A and B. Additionally, the technical solutions of the embodiments may be combined with each other on the basis that the combination is implementable by those of ordinary skill in the art. In case a combination of the technical solutions is contradictory or infeasible, such a combination is deemed inexistent and not falling within the protection scope of the present disclosure.

The foregoing are merely preferred embodiments of the present disclosure, and the scope of the present disclosure is not limited thereto. Any equivalent structure change made using the content of the specification of the present disclosure and the accompanying drawings under the inventive concept of the present disclosure, or direct/indirect application thereof in other related technical fields, shall fall within the protection scope of the present disclosure.

## Claims

1. A breathing tube, comprising:
a gas guide tube configured to convey a gas; and
a first support rib connected to an outer peripheral wall surface of the gas guide tube and
disposed around an axis of the gas guide tube;
wherein the first support rib comprises a hollow segment that is hollowed out.

2. The breathing tube according to claim 1, wherein
the first support rib is entirely hollowed out.

3. The breathing tube according to claim 1, wherein
the first support rib comprises a plurality of annular rings that are sleeved outside the gas guide tube and are arranged at intervals along a direction of the axis of the gas guide tube; or
the first support rib is spirally wound around the gas guide tube with the axis of the gas guide tube as a center axis.

4. The breathing tube according to claim 1, wherein
the first support rib is spirally wound around the gas guide tube with the axis of the gas guide tube as a center axis; and
a limiting protrusion is provided on the outer peripheral wall surface of the gas guide tube, and abuts against a spiral side edge of the first support rib along the direction of the axis of the gas guide tube.

5. The breathing tube according to claim 4, wherein
two limiting protrusions are provided on the outer peripheral wall surface of the gas guide tube, and abut against two opposite spiral side edges of the first support rib along the direction of the axis of the gas guide tube, respectively.

6. The breathing tube according to claim 5, wherein
the two limiting protrusions are spirally wound around the gas guide tube with the axis of the gas guide tube as the center axis.

7. The breathing tube according to claim 1, wherein
the hollow segment comprises a ribbed tube connected to the outer peripheral wall surface and a rib strip disposed within the ribbed tube; a side of the rib strip close to the gas guide tube is connected to an inner wall surface of a side of the ribbed tube close to the gas guide tube, and a side of the rib strip away from the gas guide tube is connected to an inner wall surface of a side of the ribbed tube away from the gas guide tube.

8. The breathing tube according to claim 7, wherein
the rib strip is disposed spirally with the axis of the gas guide tube as a center axis.

9. The breathing tube according to claim 7, wherein
a thickness of the rib strip decreases gradually along a direction away from the gas guide tube.

10. The breathing tube according to claim 7, wherein
the hollow segment comprises a plurality of rib strips that are spaced apart along a radial direction of the ribbed tube; and spacings between the rib strips increase gradually along a direction away from the gas guide tube.

11. The breathing tube according to claim 1, wherein
the gas guide tube is configured to be formed by spirally winding a strip.

12. The breathing tube according to claim 11, wherein
the strip comprises a strip body and a lug; the strip body has an inner overlapping portion on one side and an outer overlapping portion on another side along a width direction of the strip body; the inner overlapping portion and the outer overlapping portion of the strip body in a spiral shape are arranged in an overlapping manner, and the outer overlapping portion is located on a side of the inner overlapping portion away from a lumen of the gas guide tube; and
the lug is connected to the outer overlapping portion and extends along a length direction of the strip body; and the lug in the spiral shape is a limiting protrusion.

13. The breathing tube according to claim 12, wherein
one lug is provided such that the gas guide tube comprises one limiting protrusion; and along an axial direction of the gas guide tube, one side of the limiting protrusion abuts against a spiral side edge on one side of the first support rib, while another side of the limiting protrusion abuts against a spiral side edge on another side of the first support rib.

14. The breathing tube according to claim 12, wherein
two lugs are provided such that the gas guide tube comprises two limiting protrusions; and the first support rib is located between the two limiting protrusions.

15. The breathing tube according to claim 1, wherein
the gas guide tube is made of a transparent material;
and/or
the first support rib is made of a transparent material.

16. The breathing tube according to claim 1, further comprising:
a heating wire connected to the gas guide tube and/or the first support rib.

17. The breathing tube according to claim 16, wherein
the heating wire is disposed spirally with the axis of the gas guide tube as a center axis.

18. The breathing tube according to claim 16, wherein
the first support rib is spirally wound around the gas guide tube with the axis of the gas guide tube as a center axis; and
the heating wire is located between a lumen of the gas guide tube and an outer side portion of the first support rib away from the gas guide tube.

19. The breathing tube according to claim 16, wherein
the heating wire is located within the gas guide tube;
or
the heating wire is located between the gas guide tube and the first support rib;
or
the heating wire is located within the first support rib.

20. The breathing tube according to claim 16, wherein
the first support rib is entirely hollowed out, and comprises a first sidewall connected to the gas guide tube and a second sidewall away from the gas guide tube; the first sidewall and the second sidewall together define an inner cavity of the first support rib; and the heating wire is located within the first sidewall.

21. The breathing tube according to claim 16, wherein
two limiting protrusions are provided on the outer peripheral wall surface of the gas guide tube, and abut against two opposite spiral side edges of the first support rib along the direction of the axis of the gas guide tube, respectively; and
the heating wire is located between the two limiting protrusions along an axial direction of the gas guide tube.

22. The breathing tube according to claim 16, wherein
a plurality of heating wires are provided, and each of the plurality of heating wires is disposed spirally with the axis of the gas guide tube as a center axis;
or
a plurality of heating wires are provided, and each of the plurality of heating wires is disposed spirally with the axis of the gas guide tube as a center axis, with at least one of the plurality of heating wires being adapted to transfer a communication signal.

23. The breathing tube according to claim 16, wherein
the gas guide tube is configured to be formed by spirally winding a strip;
the strip comprises a strip body; the strip body has an inner overlapping portion on one side and an outer overlapping portion on another side along a width direction of the strip body; the inner overlapping portion and the outer overlapping portion of the strip body in a spiral shape are arranged in an overlapping manner, and the outer overlapping portion is located on a side of the inner overlapping portion away from a lumen of the gas guide tube; and
the heating wire is located between the inner overlapping portion and the outer overlapping portion along a radial direction of the gas guide tube.

24. The breathing tube according to claim 23, further comprising:
a second support rib connected to the outer peripheral wall surface of the gas guide tube and disposed around the axis of the gas guide tube.

25. The breathing tube according to claim 24, wherein
the second support rib is disposed spirally with the axis of the gas guide tube as a center axis; and the second support rib and the first support rib are of a dual-spiral structure.

26. The breathing tube according to claim 24, wherein
the second support rib is entirely hollowed out;
or
the second support rib and an outer peripheral wall of the gas guide tube together define a spiral cavity.

27. The breathing tube according to claim 24, wherein
a height dimension of the second support rib is smaller than a height dimension of the first support rib along the radial direction of the gas guide tube;
and/or
a width dimension of the second support rib is smaller than a width dimension of the first support rib along an axial direction of the gas guide tube.

28. The breathing tube according to claim 24, wherein
the first support rib and the gas guide tube are integrated;
and/or
the second support rib and the gas guide tube are integrated.

29. The breathing tube according to claim 24, wherein
the second support rib and the gas guide tube are integrated;
the gas guide tube is configured to be formed by spirally winding a strip;
the strip comprises a strip body and a support strip; the support strip is connected to a side of the strip body, and defines, together with the strip body, an inner cavity extending along a length direction of the strip; and
the support strip is disposed spirally to form the second support rib.

30. The breathing tube according to claim 24, wherein
the strip further comprises a lug that is connected to the strip body and located on a same side with the support strip; the lug extends along a length direction of the strip body and is spaced apart from the support strip along a width direction of the strip body; and
the lug is disposed spirally to form a limiting protrusion; and the first support rib is located between the limiting protrusion and the second support rib.

31. The breathing tube according to claim 24, wherein
the strip further comprises two lugs that are connected to the strip body and located on a same side with the support strip; the two lugs extend along a length direction of the strip body and are spaced apart from each other along a width direction of the strip body; and
the two lugs are disposed spirally to form two limiting protrusions; and the first support rib is located between the two limiting protrusions.

32. The breathing tube according to claim 1, wherein
the first support rib and the gas guide tube are integrated;
the gas guide tube is configured to be formed by spirally winding a strip;
the strip comprises a strip body and a support strip; the support strip is connected to a side of the strip body, and defines, together with the strip body, an inner cavity extending along a length direction of the strip; and
the support strip is disposed spirally to form the first support rib.

33. The breathing tube according to claim 1, meeting at least one of conditions a) to k):
a) a wall thickness d₁ of the gas guide tube meets: 0.13 mm≤d₁≤0.23 mm;
b) a wall thickness d₂ of the hollow segment meets: 0.1 mm≤d₂≤0.5 mm;
c) an inner diameter D₁ of the gas guide tube meets: 8 mm≤D₁≤28 mm;
d) along a radial direction of the gas guide tube, a height dimension L₁ of the hollow segment meets: 2.2 mm≤L₁≤2.8 mm;
e) along an axial direction of the gas guide tube, a width dimension L₂ of the hollow segment meets: 2 mm≤L₂≤6 mm;
f) the hollow segment is arranged spirally with the axis of the gas guide tube as a center axis, and a lead S of the hollow segment meets: 2 mm≤S≤8 mm;
g) the first support rib is spirally wound around the gas guide tube with the axis of the gas guide tube as the center axis; a limiting protrusion is provided on the outer peripheral wall surface of the gas guide tube, and abuts against a spiral side edge of the first support rib along a direction of the axis of the gas guide tube; and along the radial direction of the gas guide tube, a height dimension L₃ of the limiting protrusion meets: 0.3 mm≤L₃≤1.3 mm;
h) the first support rib is spirally wound around the gas guide tube with the axis of the gas guide tube as the center axis; a limiting protrusion is provided on the outer peripheral wall surface of the gas guide tube, and abuts against a spiral side edge of the first support rib along the direction of the axis of the gas guide tube; and along the axial direction of the gas guide tube, a width dimension L₄ of the limiting protrusion meets: 0.5 mm≤L₄≤1.5 mm;
i) the first support rib is arranged spirally with the axis of the gas guide tube as the center axis, and a lead of the first support rib is greater than a width dimension of the first support rib along the axial direction of the gas guide tube;
j) a second support rib is further wound around the gas guide tube; and along the radial direction of the gas guide tube, a height dimension L₅ of the second support rib meets: 1.3 mm≤L₅≤2.3 mm; and
k) a second support rib is further wound around the gas guide tube; and along the axial direction of the gas guide tube, a width dimension L₆ of the second support rib meets: 1.5 mm≤L₆≤2.5 mm.

34. A breathing tube, comprising:
a gas guide tube configured to convey a gas; and
a first support rib connected to an outer peripheral wall surface of the gas guide tube and disposed around an axis of the outer peripheral wall surface;
wherein the first support rib comprises a hollow segment; and the hollow segment and the outer peripheral wall surface of the gas guide tube together define a cavity.

35. A medical device, comprising the breathing tube according to any one of claims 1 to 33.

36. A manufacturing method, used for manufacturing the breathing tube according to any one of claims 1 to 33 and comprising steps of:
fabricating the strip;
fabricating the support strip, wherein the support strip comprises the hollow segment;
spirally winding the strip around a forming shaft to form the gas guide tube; and
spirally winding the support strip around the gas guide tube to form the first support rib.

37. The manufacturing method according to claim 36, wherein
fabricating the strip comprises: forming the strip through an extrusion molding process using a first extrusion unit;
and/or
fabricating the support strip comprises: forming the support strip through an extrusion molding process using a second extrusion unit.

38. The manufacturing method according to claim 36, wherein
spirally winding the strip around the forming shaft to form the gas guide tube comprises: disposing a first extrusion unit beside the forming shaft, and winding the formed strip around the forming shaft during a process of forming the strip by the first extrusion unit; and
spirally winding the support strip around the gas guide tube to form the first support rib comprises: disposing a second extrusion unit beside the forming shaft, and winding the formed support strip around the formed gas guide tube during a process of forming the support strip by the second extrusion unit.

39. The manufacturing method according to claim 36, wherein
spirally winding the strip around the forming shaft to form the gas guide tube further comprises: disposing a plurality of flexible spring shafts along a circumferential direction of the forming shaft, spirally winding the strip around the flexible spring shafts, and driving, by a driver, the flexible spring shafts to move along an axial direction of the forming shaft and to rotate along the circumferential direction of the forming shaft, thereby driving the strip to move spirally.

40. The manufacturing method according to claim 36, wherein
fabricating the strip comprises: integrally forming the heating wire and the strip through extrusion molding, wherein the heating wire is located within the strip;
or
fabricating the support strip comprises: integrally forming the heating wire and the support strip through extrusion molding, wherein the heating wire is located within the support strip; or
before spirally winding the support strip around the gas guide tube to form the first support rib, the method comprising: spirally winding the heating wire around the gas guide tube, spirally winding the support strip around the gas guide tube, and covering the heating wire with the support strip.

41. The manufacturing method according to claim 36, wherein
fabricating the support strip comprises: fabricating two support strips, wherein at least one of the two support strips comprises the hollow segment; and
spirally winding the support strip around the gas guide tube comprises: spirally winding the two support strips around the gas guide tube to form the dual-spiral structure, thereby forming the first support rib and the second support rib.
